# EUROPEAN PATENT APPLICATION

(11) **EP 4 198 135 A1**
(43) Date of publication of application: **21.06.2023**
(21) Application number: 21857797.1
(22) Date of filing: 28.09.2021
(51) Int. Cl.: C12N 15/866, C12N 7/00

(54) **NUCLEIC ACID CONSTRUCT FOR INCREASING ADENO-ASSOCIATED VIRUS YIELD, AND CONSTRUCTION METHOD THEREFOR**

(30) Priority: 17.08.2020 CN 202010826899; 22.09.2020 CN 202011004197
(71) Applicant: Hangzhou Geneway Biotechnology Co., Ltd., Hangzhou, Zhejiang 310018 (CN)
(72) Inventor: TAO, Yu, Hangzhou, Zhejiang 310018 (CN); WU, Haoquan, Hangzhou, Zhejiang 310018 (CN); SU, Lingling, Hangzhou, Zhejiang 310018 (CN); DANG, Ying, Hangzhou, Zhejiang 310018 (CN)
(74) Representative: D Young & Co LLP
(86) International application number: PCT/CN2021/121350
(87) International publication number: WO 2022/037710

(57) **Abstract**

A nucleic acid construct for improving an adeno-associated virus yield, and a construction method therefor. The nucleic acid construct comprises: an adeno-associated virus (AAV) element, and a polynucleotide encoding an IE protein. Said AAV element comprises a polynucleotide encoding a Cap protein, a polynucleotide encoding a Rep protein, and an AAV cis-regulatory element. The construction method comprises integrating an AAV element carrying an exogenous target gene and a polynucleotide that encodes the IE protein into to a baculovirus vector backbone. The obtained recombinant adeno-associated virus (rAAV) has a low empty capsid rate, while the rAAV yield of a single cell and a unit volume culture is increased, the production cost is reduced, and the production is easy to scale up.

## Description

### TECHNICAL FIELD

The present invention relates to the field of gene transfer vector technology, in particular to a nucleic acid construct for increasing adeno-associated virus (AAV) yield and construction method therefor.

### TECHNICAL BACKGROUND

Recombinant adeno-associated virus (rAAV) is a type of gene transfer vector widely used in basic research and clinical gene therapy. Because of its clinical safety, broad host range, low immunogenicity, and long-lasting expression of exogenous genes *in vivo,* rAAV has been considered one of the most promising gene transfer vectors and widely used in gene therapy and vaccine research around the world. In recent years, more and more investigators start using rAAV for preclinical studies in large animals and clinical trials. AAV genome comprises three key elements: ITR sequences, coding genes for nonstructural protein Rep, and coding genes for structural protein Cap.

Baculovirus/insect cell system is often used for production of recombinant proteins. Compared with the production using the standard triple-transfection method in HEK293 cells, insect cells can be cultured at high density, serum-free suspension culture, and can be easily scaled up. And, the production method using baculovirus-infected cells is more stable batch to batch. Since 2002, investigators have begun to use baculovirus/insect cell system for the production of rAAVs. However, due to various reasons associated with the production in insect cells, e.g., the ratio of Cap, Rep, and AAV vector genomic DNAs is not easy to reach the ideal ratio, the packaging efficiency of AAV is not high. And, the rAAV vectors that are packaged by traditional methods have a higher empty capsid rate (11 %-34%) (Benskey et al., 2016). Empty AAV capsid is an impurity that must be removed in the preparation of clinical-grade AAV vectors. Because the physical and chemical properties of empty AAV and AAV carrying target gene are very similar, purification is very difficult.

### DESCRIPTION OF THE INVENTION

In view of the above-mentioned shortcomings of the prior art, the purpose of the present invention is to provide a nucleic acid construct and its construction method for improving the yield and quality of the rAAVs, so as to solve the problems in the prior art.

In order to achieve the above purpose and other related purposes, the nucleic acid construct provided by the first aspect of the present invention includes: AAV elements, polynucleotides encoding IE protein, and polynucleotides encoding recombination homologous region of baculovirus, and the AAV elements include a polynucleotide encoding Cap proteins, a polynucleotide encoding Rep proteins, and an AAV cis-acting element.

In some embodiments, the IE protein is encoded by one or more genes of Acie0, Acie01, Acie02, and/or, the recombination homologous region of baculovirus is selected from one or more of hr1, hr2, hr3, hr4, and hr5.

In some embodiments, the nucleic acid construct further includes a promoter of the IE protein gene. In some embodiments, the promoter of the IE protein gene is selected from one or more of Gp64, pH, p6.9, or p10.

In some embodiments, the nucleic acid construct further includes a baculovirus promoter. In some embodiments, the baculovirus promoter is linked to a recombination homologous region of baculovirus. In some embodiments, the baculovirus promoter is preferably one or more of pH, Gp64, p6.9, or p10.

In some embodiments, the AAV cis-acting element is selected from ITR sequences.

In some embodiments, the nucleic acid construct further includes an exogenous gene of interest. In some embodiments, the exogenous gene of interest is embedded in between AAV elements.

In some embodiments, the structure of the nucleic acid construct includes: IE gene expression cassette-Cap gene expression cassette-hr1-ITR-exogenous target gene expression cassette-ITR-Rep gene expression cassette. In some embodiments, the structure of the nucleic acid construct includes: IE gene expression cassette-Cap gene expression cassette-ITR-exogenous target gene expression cassette-ITR-Rep gene expression cassette.

In some embodiments, the nucleotide sequence of the nucleic acid construct comprises SEQ ID NO. 1, or comprises nucleotide sequences that share at least 75%, 80%, 85%, 90%, 95%, 96 %, 97%, 98%, or 99% homology with SEQ ID NO. 1. In some embodiments, the nucleotide sequence of the nucleic acid construct is shown in SEQ ID NO. 1.

In some embodiments, the nucleic acid construct is an AAV vector or a recombinant baculovirus vector. In some embodiments, the nucleic acid construct is an AAV vector. In some embodiments, the nucleic acid construct is a recombinant baculovirus vector. In some embodiments, the ecombinant baculovirus vector is preferably a recombinant baculovirus shuttle vector.

The second aspect of the present invention provides a recombinant baculovirus, and the recombinant baculovirus is obtained by constructing any one of the nucleic acid constructs through the baculovirus system, or is obtained by constructing nucleic acid constructs by including any element of the nucleic acid constructs through the baculovirus system.

The third aspect of the present invention provides an adeno-associated virus, and said AAV is obtained after infection of cells with any one of the recombinant baculoviruses and packaging.

The fourth aspect of the present invention provides a cell line, which is a cell line infected by any one of the recombinant baculoviruses.

The fifth aspect of the present invention provides an AAV vector system, which includes a baculovirus system and the nucleic acid construct.

The sixth aspect of the present invention provides the construction method of the nucleic acid construct, and the construction method includes integrating the AAV elements carrying the exogenous gene of interest, the polynucleotide encoding the IE protein, and the polynucleotide encoding the recombination homologous region of baculovirus into the backbone of the baculovirus vector.

The construction method includes one or more (e.g., two or three) of the following features:
1) The AAV elements include the polynucleotide encoding Cap proteins, the polynucleotide encoding Rep proteins, and AAV cis-acting elements, e.g., the AAV cis-acting elements are preferably ITR sequences;
2) The polynucleotide encoding the IE protein is selected from one or more of Acie0, Acie01, or Acie02;
3) The backbone of the baculovirus vector is selected from one of pFastBacdual, pFastBac1, pFastBacHTA, pFastBacHTB, or pFastBacHTC; or
4) The recombination homologous region of the baculovirus is selected from one or more of hr1, hr2, hr3, hr4, or hr5.

The seventh aspect of the present invention provides a production method for increasing the yield of AAV, and the production method comprises the following steps: infecting insect cell lines with the recombinant baculovirus.

As mentioned above, the nucleic acid construct and its construction method of the present invention for improving the yield of AAV have at least the following beneficial effects:
1) The present invention significantly reduces the expression of the target gene inserted in the baculovirus vector in insect cells, reduces the impact of target gene expression on insect cells; improving the growth index of production cells makes it more conducive to the packaging and production of rAAV.
2) Compared with the production using traditional adherent 293 and 293T cells, as well as conventional production methods using baculovirus, this optimized vector construction method significantly improves rAAV production of single cells and unit volume cultures, and reduces production costs, and the production using this method can be scaled up.
3) A wide range of applications, including the production of AAV gene therapy vectors of various scales and types.

### Description of Drawings and Figures

Figure 1 is a schematic diagram showing the structure of the pFBd-Cap-ITR-Rep vector of the present invention.
Figure 2 is a schematic diagram showing the map of the pFBd-Cap-ITR-Rep vector of the present invention.
Figure 3 is a schematic diagram showing the structure of the pFBd-IE-hr1Cap-ITR-Rep vector of the present invention.
Figure 4 is a schematic diagram showing the map of the pFBd-IE-hr1Cap-ITR-Rep vector of the present invention.
Figure 5 shows the comparison of EGFP expression levels in Sf9 cells 2-4 days after infection with BV-Cap-ITR-Rep and BV-IE-hr1Cap-ITR-Rep of the present invention.
Figure 6 shows Western blotting detection of lysates of cells infected with two baculoviruses of the present invention using AAV Cap monoclonal antibody.
Figure 7 shows the electron microscopy examination of rAAV2 of the present invention (titer 2.0E+13 V G/mL).
Figure 8 shows the fluorescence observation of 293T cells 2 days after transduction with rAAV2 packaged with BV-Cap-ITR-Rep and BV-IE-hr1Cap-ITR-Rep of the present invention.

### Detailed Description

The present invention is based, at least in part, on the discovery that a nucleic acid construct comprising AAV elements, a polynucleotide encoding an IE protein, and a polynucleotide encoding a recombination homologous region of baculovirus can significantly control the expression of a gene of interest inserted in the baculovirus in its host insect cells, improve the state of cells and the packaging efficiency of AAV, thereby greatly increasing the yield of AAV. The present invention can unexpectedly reduce the empty capsid rate of rAAV (for example, even if the expression level of Cap is increased, it will not lead to an increase in empty capsid), produce rAAV with better infectivity, and significantly improve the yield of rAAV of single cells and unit volume cultures, therefore is suitable for large-scale production of various AAV gene therapy vectors.

Unless otherwise defined below, all technical and scientific terms mentioned herein have the meanings commonly understood by those skilled in the art to which this invention belongs.

The term "nucleic acid construct" refers to an artificially constructed nucleic acid segment that can be introduced into target cells or tissues, and the nucleic acid construct can be a lentiviral vector or an AAV vector, which includes a vector backbone, i.e., the empty vector and expression framework.

The term "vector" refers to a nucleic acid fragment or polynucleotide fragment used to introduce or transfer one or more nucleic acids or one or more polynucleotides into target cells or tissues. Typically, vectors are used to introduce foreign DNA into another cell or tissue. The vector may contain a bacterial resistance gene for growth in bacteria and a promoter for expressing a protein of interest in an organism. DNA can be produced *in vitro* by PCR or any other suitable technique or techniques known to those skilled in the art.

The first aspect of the present invention provides a nucleic acid construct, said nucleic acid construct comprising: AAV elements and a polynucleotide encoding an IE protein, and the AAV elements comprising a polynucleotide encoding Cap proteins, a polynucleotide encoding Rep proteins, and AAV cis-acting elements.

In one embodiment, the nucleic acid construct further includes a polynucleotide encoding a recombination homologous region of baculovirus.

In some embodiments, the nucleotide sequence of the nucleic acid construct comprises SEQ ID NO.1, or comprises nucleotide sequences that share at least 75%, 80%, 85%, 90%, 95%, 96 %, 97%, 98%, or 99% homology to SEQ ID NO.1. In some embodiments, the nucleotide sequence of the nucleic acid construct is shown in SEQ ID NO.1.

SEQ ID NO: 1:
TTCTCTGTCACAGAATGAAAATTTTTCTGTCATCTCTTCGTTATTAATGTTTGTAATTGACTGAA
TATCAACGCTTATTTGCAGCCTGAATGGCGAATGGGACGCGCCCTGTAGCGGCGCATTAAGCG
CGGCGGGTGTGGTGGTTACGCGCAGCGTGACCGCTACACTTGCCAGCGCCCTAGCGCCCGCT
CCTTTCGCTTTCTTCCCTTCCTTTCTCGCCACGTTCGCCGGCTTTCCCCGTCAAGCTCTAAATC
GGGGGCTCCCTTTAGGGTTCCGATTTAGTGCTTTACGGCACCTCGACCCCAAAAAACTTGATT
AGGGTGATGGTTCACGTAGTGGGCCATCGCCCTGATAGACGGTTTTTCGCCCTTTGACGTTGG

AGTCCACGTTCTTTAATAGTGGACTCTTGTTCCAAACTGGAACAACACTCAACCCTATCTCGG
TCTATTCTTTTGATTTATAAGGGATTTTGCCGATTTCGGCCTATTGGTTAAAAAATGAGCTGATT
TAACAAAAATTTAACGCGAATTTTAACAAAATATTAACGTTTACAATTTCAGGTGGCACTTTTC
GGGGAAATGTGCGCGGAACCCCTATTTGTTTATTTTTCTAAATACATTCAAATATGTATCCGCTC
ATGAGACAATAACCCTGATAAATGCTTCAATAATATTGAAAAAGGAAGAGTATGAGTATTCAAC
ATTTCCGTGTCGCCCTTATTCCCTTTTTTGCGGCATTTTGCCTTCCTGTTTTTGCTCACCCAGAA
ACGCTGGTGAAAGTAAAAGATGCTGAAGATCAGTTGGGTGCACGAGTGGGTTACATCGAACT
GGATCTCAACAGCGGTAAGATCCTTGAGAGTTTTCGCCCCGAAGAACGTTTTCCAATGATGAG
CACTTTTAAAGTTCTGCTATGTGGCGCGGTATTATCCCGTATTGACGCCGGGCAAGAGCAACTC
GGTCGCCGCATACACTATTCTCAGAATGACTTGGTTGAGTACTCACCAGTCACAGAAAAGCAT
CTTACGGATGGCATGACAGTAAGAGAATTATGCAGTGCTGCCATAACCATGAGTGATAACACT
GCGGCCAACTTACTTCTGACAACGATCGGAGGACCGAAGGAGCTAACCGCTTTTTTGCACAA
CATGGGGGATCATGTAACTCGCCTTGATCGTTGGGAACCGGAGCTGAATGAAGCCATACCAAA
CGACGAGCGTGACACCACGATGCCTGTAGCAATGGCAACAACGTTGCGCAAACTATTAACTG
GCGAACTACTTACTCTAGCTTCCCGGCAACAATTAATAGACTGGATGGAGGCGGATAAAGTTG
CAGGACCACTTCTGCGCTCGGCCCTTCCGGCTGGCTGGTTTATTGCTGATAAATCTGGAGCCG
GTGAGCGTGGGTCTCGCGGTATCATTGCAGCACTGGGGCCAGATGGTAAGCCCTCCCGTATCG
TAGTTATCTACACGACGGGGAGTCAGGCAACTATGGATGAACGAAATAGACAGATCGCTGAGA
TAGGTGCCTCACTGATTAAGCATTGGTAACTGTCAGACCAAGTTTACTCATATATACTTTAGATT
GATTTAAAACTTCATTTTTAATTTAAAAGGATCTAGGTGAAGATCCTTTTTGATAATCTCATGAC
CAAAATCCCTTAACGTGAGTTTTCGTTCCACTGAGCGTCAGACCCCGTAGAAAAGATCAAAG
GATCTTCTTGAGATCCTTTTTTTCTGCGCGTAATCTGCTGCTTGCAAACAAAAAAACCACCGCT
ACCAGCGGTGGTTTGTTTGCCGGATCAAGAGCTACCAACTCTTTTTCCGAAGGTAACTGGCTT
CAGCAGAGCGCAGATACCAAATACTGTCCTTCTAGTGTAGCCGTAGTTAGGCCACCACTTCAA
GAACTCTGTAGCACCGCCTACATACCTCGCTCTGCTAATCCTGTTACCAGTGGCTGCTGCCAGT
GGCGATAAGTCGTGTCTTACCGGGTTGGACTCAAGACGATAGTTACCGGATAAGGCGCAGCG
GTCGGGCTGAACGGGGGGTTCGTGCACACAGCCCAGCTTGGAGCGAACGACCTACACCGAA
CTGAGATACCTACAGCGTGAGCATTGAGAAAGCGCCACGCTTCCCGAAGGGAGAAAGGCGG
ACAGGTATCCGGTAAGCGGCAGGGTCGGAACAGGAGAGCGCACGAGGGAGCTTCCAGGGGG
AAACGCCTGGTATCTTTATAGTCCTGTCGGGTTTCGCCACCTCTGACTTGAGCGTCGATTTTTG
TGATGCTCGTCAGGGGGGCGGAGCCTATGGAAAAACGCCAGCAACGCGGCCTTTTTACGGTT
CCTGGCCTTTTGCTGGCCTTTTGCTCACATGTTCTTTCCTGCGTTATCCCCTGATTCTGTGGATA
ACCGTATTACCGCCTTTGAGTGAGCTGATACCGCTCGCCGCAGCCGAACGACCGAGCGCAGC
GAGTCAGTGAGCGAGGAAGCGGAAGAGCGCCTGATGCGGTATTTTCTCCTTACGCATCTGTG

CGGTATTTCACACCGCAGACCAGCCGCGTAACCTGGCAAAATCGGTTACGGTTGAGTAATAAA
TGGATGCCCTGCGTAAGCGGGTGTGGGCGGACAATAAAGTCTTAAACTGAACAAAATAGATCT
AAACTATGACAATAAAGTCTTAAACTAGACAGAATAGTTGTAAACTGAAATCAGTCCAGTTAT
GCTGTGAAAAAGCATACTGGACTTTTGTTATGGCTAAAGCAAACTCTTCATTTTCTGAAGTGC
AAATTGCCCGTCGTATTAAAGAGGGGCGTGGCCAAGGGCATGGTAAAGACTATATTCGCGGCG
TTGTGACAATTTACCGAACAACTCCGCGGCCGGGAAGCCGATCTCGGCTTGAACGAATTGTTA
GGTGGCGGTACTTGGGTCGATATCAAAGTGCATCACTTCTTCCCGTATGCCCAACTTTGTATAG
AGAGCCACTGCGGGATCGTCACCGTAATCTGCTTGCACGTAGATCACATAAGCACCAAGCGCG
TTGGCCTCATGCTTGAGGAGATTGATGAGCGCGGTGGCAATGCCCTGCCTCCGGTGCTCGCCG
GAGACTGCGAGATCATAGATATAGATCTCACTACGCGGCTGCTCAAACCTGGGCAGAACGTAA
GCCGCGAGAGCGCCAACAACCGCTTCTTGGTCGAAGGCAGCAAGCGCGATGAATGTCTTACT
ACGGAGCAAGTTCCCGAGGTAATCGGAGTCCGGCTGATGTTGGGAGTAGGTGGCTACGTCTC
CGAACTCACGACCGAAAAGATCAAGAGCAGCCCGCATGGATTTGACTTGGTCAGGGCCGAGC
CTACATGTGCGAATGATGCCCATACTTGAGCCACCTAACTTTGTTTTAGGGCGACTGCCCTGCT
GCGTAACATCGTTGCTGCTGCGTAACATCGTTGCTGCTCCATAACATCAAACATCGACCCACG
GCGTAACGCGCTTGCTGCTTGGATGCCCGAGGCATAGACTGTACAAAAAAACAGTCATAACA
AGCCATGAAAACCGCCACTGCGCCGTTACCACCGCTGCGTTCGGTCAAGGTTCTGGACCAGT
TGCGTGAGCGCATACGCTACTTGCATTACAGTTTACGAACCGAACAGGCTTATGTCAACTGGG
TTCGTGCCTTCATCCGTTTCCACGGTGTGCGTCACCCGGCAACCTTGGGCAGCAGCGAAGTC
GAGGCATTTCTGTCCTGGCTGGCGAACGAGCGCAAGGTTTCGGTCTCCACGCATCGTCAGGC
ATTGGCGGCCTTGCTGTTCTTCTACGGCAAGGTGCTGTGCACGGATCTGCCCTGGCTTCAGGA
GATCGGTAGACCTCGGCCGTCGCGGCGCTTGCCGGTGGTGCTGACCCCGGATGAAGTGGTTC
GCATCCTCGGTTTTCTGGAAGGCGAGCATCGTTTGTTCGCCCAGGACTCTAGCTATAGTTCTAG
TGGTTGGCCTACGTACCCGTAGTGGCTATGGCAGGGCTTGCCGCCCCGACGTTGGCTGCGAGC
CCTGGGCCTTCACCCGAACTTGGGGGTTGGGGTGGGGAAAAGGAAGAAACGCGGGCGTATT
GGTCCCAATGGGGTCTCGGTGGGGTATCGACAGAGTGCCAGCCCTGGGACCGAACCCCGCGT
TTATGAACAAACGACCCAACACCCGTGCGTTTTATTCTGTCTTTTTATTGCCGTCATAGCGCGG
GTTCCTTCCGGTATTGTCTCCTTCCGTGTTTCAGTTAGCCTCCCCCATCTCCCGGTACCGCATGC
CTTAGTTGAACTCGAACTTCTTGTACTTGCAGTTGAGCTTTTGCTCGGCGAATGTGATGGCGT
CGGAGAGTGGCACCAGTCCCTGCAGGATCAAGGCCAAGAGCTTCAGCAAGTTGTTGTGCAGT
GTAGTTGACTCGCGACGGTTGACCTTGCCGATCACGAACATGTGGTGCTTGAATCTGTTGTAC
TTTTGGATGACCTGGCTCACATCAACGTCCTTGATTTCGCCAGAGATCCAGTAGAACTCCTTGT
TCTTCTTAGCGATGGTCAGCCTTTCCTCGTTCTTGAAGGACAACACGATGAAGTTGTGACCCT
TAACGTTATCGACGTTTTGAGTCAAGTACTGCTCAACGATGTGCATGCTTCCGTCTTCCTTCTT

GACCTTCTTGAGGTTCTCGGCGTTGTTGTTAGAAGCGATGTTGTCGTGGTACTTGTAGTTGTTG
AAGAGCAGGTTAGCGACGGACGAGTACTTGTATGTCAGAGTGCTCTTCTTGTTCACGATGAGG
TTCAGGTTATCAACGACGTACTTGTTGGGAGGGTTGTCCGGGAATTGGACGGACTCCGAGTAC
TTGAGGATCTGGGAAACGTATGGCGAGACGAAGAAGTTGTTAGAGGCGGTTTCGATCTCGTT
TGATTCCTTGCGGCTCAGCATGATGGGCAAAGTGAAGAGGTTCTTGTCCTGGTACATCTCGTA
CAACTTCGACAAGAGGAATCCACACTTACGCTCGCCGAGTGATTGCAGCAAGGTCACGAAGG
TTGTCTGAGCGTAGTACATATCGAGGTTGAAGTAGGAGGTCAGAGCGGCCTTGAATGTGTGGT
GGACGTCCACGAAGTGACACTTCTTGCAGTTTTGAGCGGCGGTCTCATCGTTGCAGACGTCC
TGTGAGTGTGGGATTTCGATGCCAGTCTCCTTAACCAGGTTGTAGCTGATCATGAAGCGGATC
TTATCGAAGGTCACAACGAACACACGGTTGTCAACCATGTAGTAGTTGTTTGTGTACTCGTAG
ACCACGTTAGAGACGTACTTGGCGAAGATGATTTCGAAAGGCTTCACCTCGGACTTCTTAACG
ACGAACATGTAGTAACCGGTTTCAGACATGTGATCGGAGAATCTGTTCGAGTTGTAGTCGTTA
TCGTCGAACCTCATCAGGTAGGGGGCGAAGTCGTTTGTGAAGTAGTGAGTGATCTCCTGGGT
GGAAGCCACTGTACAGATGTTAGTGTTGTGGTTGATGGTTTGTTCCAGTGTAGCGCATGACTG
GATGGTGCTCTTCTTGTACTTAGGTCTCAACTTGATCTTGTTGAATTGACCCACAACTCCCTGG
GAGTTATCCAGGTACTCGTCCAACTTCCTCTTGGTGCCTGTGGCCGACGGCTGGTTGACACCA
GCAGAGTGTTCGAAGGACTCGGCGTGGTAAGCTGAGCTAGGAGAGGGTTGTTCGACCACTG
GCTGTTCGAGTGACTCGCTGTAGTAGGCAGAGGACACAGCTTCCTCCAGGTTGTCAGTGGTC
TTGAGCAGACACTCCACCAAATCGTTGTCAGTGAGCGAGTTAACTGAGGCGAGGAAGTTGCT
AGCGGCAGCGGTTTCAGAGTCGGAGATGACAGTATCAGCTCCGTCCGGGGTTGGGTGGTTGT
AGTAAGACAAGTAATCGTTAGGTTGCTTGTCGCAGAACTCCGAGTATGAGTTGTCGAAGCTAG
CACGAGAGGGAGTGCTGGCAGAGGTGTAGGAAGCGTTGAAGTTGATTTGAGTCATGGTGACC
TGGTTGTTCACGATCTTATCGCCACCTGTGTCCACCTGCAGTTGCTGGGCCTCAGCGCAGGCT
GAAGTGGCCTCACAGGAGTAGGGGCTGGAAGCACAGTTGGAAGTCATGATGTTTTCTTGGAC
GTTCAGGACGTGGCTGGATGTACGGATCATGCGCCCGATGGTGGGACGGTATGAATAATCCGG
AATATTTATAGGTTTTTTTATTACAAAACTGTTACGAAAACAGTAAAATACTTATTTATTTGCGA
GATGGTTATCATTTTAATTATCTCCATGATCGGATCTCCTAGGCTCAAGCAGTGATCAGATCCAG
ACATGATAAGATACATTGATGAGTTTGGACAAACCACAACTAGAATGCAGTGAAAAAAATGCT
TTATTTGTGAAATTTGTGATGCTATTGCTTTATTTGTAACCATTATAAGCTGCAATAAACAAGTT
AACAACAACAATTGCATTTGTCTAGTACTTCTCGACGGTACCGCATGCTATGCATCAGCTGCTA
GCACCATGGCTCGAGTTACAGATTACGAGTCAGGTATCTGGTGCCAATGGGGCGAGGCTCTGA
ATACACGCCATTAGTGTCCACAGTAAAGTCCACATTAACAGACTTGTTGTAGTTGGAAGTGTA
CTGAATTTCGGGATTCCAGCGTTTGCTGTTTTCCTTCTGCAGCTCCCACTCGATCTCCACGCTG
ACCTGTCCCGTGGAGTACTGTGTGATGAAGGAAGCAAACTTTGCCGCACTGAAGGTGGTCGA

AGGATTCGCAGGTACCGGGGTGTTCTTGATGAGAATCTGTGGAGGAGGGTGTTTAAGTCCGA
ATCCACCCATGAGGGGAGAGGGGTGAAAATGTCCGTCCGTGTGTGGAATCTTTGCCCAGATG
GGCCCCTGAAGGTACACATCTCTGTCCTGCCAGACCATGCCTGGAAGAACGCCTTGTGTGTTG
ACATCTGCGGTAGCTGCTTGTCTGTTGCCTCTCTGGAGGTTGGTAGATACAGAACCATACTGCT
CCGTAGCCACGGGATTGGTTGTCCTGATTTCCTCTTCGTCTGTAATCATGACCTTTTCAATGTC
CACATTTGTTTTCTCTGAGCCTTGCTTCCCAAAGATGAGAACCCCGCTCTGAGGAAAAAACTT
TTCTTCATCGTCCTTGTGGCTTGCCATCGCCGGGCCCGGATTCACCAGAGAGTCTCTGCCATTG
AGGTGGTACTTGGTAGCTCCAGTCCACGAGTATTCACTGTTGTTGTTATCCGCAGATGTCTTTG
ATACTCGCTGCTGGCGGTAACAGGGTCCAGGAAGCCAGTTCCTAGACTGGTCCCGAATGTCA
CTCGCTCCGGCCTGAGAAAACTGAAGCCTTGACTGCGTGGTGGTTCCACTTGGAGTGTTTGT
TCTGCTCAAGTAATACAGGTACTGGTCGATGAGAGGATTCATGAGACGGTCCAGACTCTGGCT
GTGAGCGTAGCTGCTGTGGAAAGGAACGTCCTCAAAAGTGTAGCTGAAGGTAAAGTTGTTTC
CGGTACGCAGCATCTGAGAAGGAAAGTACTCCAGGCAGTAAAATGAAGAGCGTCCTACTGCC
TGACTCCCGTTGTTCAGGGTGAGGTATCCATACTGTGGCACCATGAAGACGTCTGCTGGGAAC
GGCGGGAGGCATCCTTGATGCGCCGAGCCGAGGACGTACGGGAGCTGGTACTCCGAGTCAGT
AAACACCTGAACCGTGCTGGTAAGGTTATTGGCAATCGTCGTCGTACCGTCATTCTGCGTGAC
CTCTTTGACTTGAATGTTAAAGAGCTTGAAGTTGAGTCTCTTGGGTCGGAATCCCCAGTTGTT
GTTGATGAGTCTTTGCCAGTCACGTGGTGAAAAGTGGCAGTGGAATCTGTTGAAGTCAAAAT
ACCCCCAAGGGGTGCTGTAGCCAAAGTAGTGATTGTCGTTCGAGGCTCCTGATTGGCTGGAA
ATTTGTTTGTAGAGGTGGTTGTTGTAGGTGGGCAGGGCCCAGGTTCGGGTGCTGGTGGTGATG
ACTCTGTCGCCCATCCATGTGGAATCGCAATGCCAATTTCCCGAGGAATTACCCACTCCGTCG
GCGCCCTCGTTATTGTCTGCCATTGGTGCGCCACTGCCTGTAGCCATCGTATTAGTTCCCAGAC
CAGAGGGGGCTGCTGGTGGCTGTCCGAGAGGCTGGGGGTCAGGTACTGAGTCTGCGTCTCCA
GTCTGACCAAAATTCAATCTTTTTCTTGCAGGCTGCTGGCCCGCCTTTCCGGTTCCCGAGGAG
GAGTCTGGCTCCACAGGAGAGTGCTCTACCGGCCTCTTTTTTCCCGGAGCCGTCTTAACAGGT
TCCTCAACCAGGCCCAGAGGTTCAAGAACCCTCTTTTTCGCCTGGAAGACTGCTCGTCCGAG
GTTGCCCCCAAAAGACGTATCTTCTTTAAGGCGCTCCTGGAACTCCGCGTCGGCGTGGTTGTA
CTTGAGGTAAGGGTTGTCTCCGCTGTCGAGCTGCCGGTCGTAGGCTTTGTCGTGCTCGAGGGC
AGCGGCGTCTGCCTCGTTCACCGGCTCTCCCTTGTCCAGTCCGTTGAAGGGTCCGAGGTACTT
GTACCCGGGAAGCACAAGGCCCCTGCTGTCGTCCTTATGCCGCTCTGCGGGCTTTGGTGGTGG
TGGGCCGGGTTTGAGCTTCCACCACTGTCTTATTCCTTCAGAGAGAGTGTCCTCGAGCCAATC
GGGTAGATAACCGTCGGCAGCCGTGGCGGCTCAAGTCTTCGTCGAGTGATTGTAAATAAAATG
TAATTTACAGTATAGTATTTTAATTAATATACAAATGATTTGATAATAATTCTTATTTAACTATAATA
TATTGTGTTGGGTTGAATTAAAGGTCCGTATCTCCAGACTGCAGTTTAAATTGTGTAATTTATGT

AGCTGTAATTTTTACCTTATTAATATTTTTTACGCTTTGCATTCGACGACTGAACTCCCAAATATA
TGTTTAACTCGTCTTGGTCGTTTGAATTTTTGTTGCTGTGTTTCCTAATATTTTCCATCACCTTA
AATATGTTATTGTAATCCTCAATGTTGAACTTGCAATTGGACACGGCATAGTTTTCCATAGTCGT
GTAAAACATGGTATTGGCTGCATTGTAATACATCCGACTGAGCGGGTACGGATCTATGTGTTTG
AGCAGCCTGTTCAAAAACTCTGCATCGTCGCAAAACGGAATTTGGTCACGATCCCGGTCCGA
AGCGCGCGGAATTCAAAGCGCTACGTCAGCGACGTCATCGATTATTGCTCCAATACTAGGGTT
TTGTTTTACTATCTGTTCTGCGGTCCACACATCTTCTTCGCTAGTCACGTACGCAAACAACTCG
GGCCGCAAAGGTGTTTTAAAACATATCAGATTAGAATCTTTTATAACTTGGCCGCATTGTAAAT
AGTTGTGCCAACGCGCGGGAAACATTTTACACGTGCTTTCAATCTTATCTCATAAACGCAACT
AAATATGATCCTTCAACTCGCTTTACGAGTAGAATTCTACTTGTAACGCACGATTAATTATGACT
CATAAGCTGATGTCATGTTTTGCACACGGCTCATAACCGAACTCGCTTTACGAGTAGAATTCTA
CGCGTAAAACACAATCAAGTATGAGTCATAAGCTGATGTCATGTTTTGCACACGGCTCATAAC
CGAACTGGCTTTACGAGTAGAATTCTACTTGTAACGCACGATCAGTGGATGATGTCATAAACTT
ATCTCATGTTTTGCACACGGCTCATAACTTGAGTCATAAGCTGATATCAGAAGATTATTAACGT
GTTTAGGTATGACTCATTTGTTTTCAAAACTGAACTCGCTTTACGGATAGAATTCTACTTGTAA
AACACAATCAGGGGATGATGTCATTATACAAATGATGTCATTTGTTTTTCAAAACTAAACTCGC
TTTACGGGTAGAATTCTACTTGTAAAACACAATCGAGGGATAATGTCATTTGTTATTCTCAAATA
TGATGTCATTTAAATTTTTATTATGCAAGAATTGTATGTTGCTGTGTCATTTATGAATGTTTTATC
GACAACTTGTTTATTTGTTTCATTGACTTGTTGTTGTTCGTGATTATGATTAATTATAAATCTTTT
GTTGGGGTCAACATCGATGTATACGCCGCTACAGGGCGCGTACTATGGTTGCTTTGACGTATGC
GGTGTGAAATACCGCACAGATGCGTAAGGAGAAAATACCGCATCAGGCGCCCCTGCAGGCAG
CTGCGCGCTCGCTCGCTCACTGAGGCCGCCCGGGCAAAGCCCGGGCGTCGGGCGACCTTTGG
TCGCCCGGCCTCAGTGAGCGAGCGAGCGCGCAGAGAGGGAGTGGCCAACTCCATCACTAGG
GGTTCCTACGCGTTAGTTATTAATAGTAATCAATTACGGGGTCATTAGTTCATAGCCCATATATG
GAGTTCCGCGTTACATAACTTACGGTAAATGGCCCGCCTGGCTGACCGCCCAACGACCCCCGC
CCATTGACGTCAATAATGACGTATGTTCCCATAGTAACGCCAATAGGGACTTTCCATTGACGTC
AATGGGTGGAGTATTTACGGTAAACTGCCCACTTGGCAGTACATCAAGTGTATCATATGCCAAG
TACGCCCCCTATTGACGTCAATGACGGTAAATGGCCCGCCTGGCATTATGCCCAGTACATGACC
TTATGGGACTTTCCTACTTGGCAGTACATCTACGTATTAGTCATCGCTATTACCATGGTGATGCG
GTTTTGGCAGTACATCAATGGGCGTGGATAGCGGTTTGACTCACGGGGATTTCCAAGTCTCCA
CCCCATTGACGTCAATGGGAGTTTGTTTTGGCACCAAAATCAACGGGACTTTCCAAAATGTCG
TAACAACTCCGCCCCATTGACGCAAATGGGCGGTAGGCGTGTACGGTGGGAGGTCTATATAAG
CAGAGCTGGTTTAGTGAACCGTCAGATCCGCTAGCGCTACCGGTCGCCACCGTCGACGTTTAT
TTGTGAAATTTGTGATGCTATTGCTTTATTTGTAACCATCTAGCTTTATTTGTGAAATTTGTGATG

CTATTGCTTTATTTGTAACCATTATAAGCTGCAATAAACAAGTTAACAACAACAATTGCATTCAT
TTTATGTTTCAGGTTCAGGGGGAGATGTGGGAGGTTTTTTAAAGTTTAAACAGGAACCCCTAG
TGATGGAGTTGGCCACTCCCTCTCTGCGCGCTCGCTCGCTCACTGAGGCCGGGCGACCAAAG
GTCGCCCGACGCCCGGGCGGCCTCAGTGAGCGAGCGAGCGCGCAGCTGCCTGCAGGACATG
TGAGCAAAAGGCCAGCAAAAGGCCAGGAACCGTAAAAAGGCCGCGTTGCTGGCGTTTTTCC
ATAGGCTCCGCCCCCCTGACGAGCATCACAAAAATCGACGCTCGCGGCCGCTCTAGAGATCAT
GGAGATAATTAAAATGATAACCATCTCGCAAATAAATAAGTATTTTACTGTTTTCGTAACAGTTT
TGTAATAAAAAAACCTATAAATATTCCGGATTATTCATACCGTCCCACCATCGGGCGCGGATCC
GCCGCCCTGGCGGGGTTTTACGAGATTGTGATTAAGGTCCCCAGCGACCTTGACGAGCATCTG
CCCGGCATTTCTGACAGCTTTGTGAACTGGGTGGCCGAGAAGGAGTGGGAGTTGCCGCCAGA
TTCTGACTTGGATCTGAATCTGATTGAGCAGGCACCCCTGACCGTGGCCGAGAAGCTGCAGC
GCGACTTTCTGACGGAGTGGCGCCGTGTGAGTAAGGCCCCGGAGGCCCTTTTCTTTGTGCAAT
TTGAGAAGGGAGAGAGCTACTTCCACTTACACGTGCTCGTGGAAACCACCGGGGTGAAATCC
TTAGTTTTGGGACGTTTCCTGAGTCAGATTCGCGAAAAACTGATTCAGAGAATTTACCGCGGG
ATCGAGCCGACTTTGCCAAACTGGTTCGCGGTCACAAAGACCAGAAACGGCGCCGGAGGCG
GGAACAAGGTGGTGGACGAGTGCTACATCCCCAATTACTTGCTCCCCAAAACCCAGCCTGAG
CTCCAGTGGGCGTGGACTAATTTAGAACAGTATTTAAGCGCCTGTTTGAATCTCACGGAGCGT
AAACGGTTGGTGGCGCAGCATCTGACGCACGTGTCGCAGACGCAGGAGCAGAACAAAGAGA
ATCAGAATCCCAATTCTGACGCGCCGGTGATCAGATCAAAAACTTCAGCCAGGTACATGGAGC
TGGTCGGGTGGCTCGTGGACAAGGGGATTACCTCGGAGAAGCAGTGGATACAGGAGGACCA
GGCCTCATACATCTCCTTCAATGCGGCCTCCAACTCGCGGTCCCAAATCAAGGCTGCCTTGGA
CAATGCGGGAAAGATTATGAGCCTGACTAAAACCGCCCCCGACTACCTGGTGGGCCAGCAGC
CCGTGGAGGACATTTCCAGCAATCGGATTTATAAAATTTTGGAACTAAACGGGTACGATCCCC
AATATGCGGCTTCCGTCTTTCTGGGATGGGCCACGAAAAAGTTCGGCAAGAGGAACACCATC
TGGCTGTTTGGGCCTGCAACTACCGGGAAGACCAACATCGCGGAGGCCATAGCCCACACTGT
GCCCTTCTACGGGTGCGTAAACTGGACCAATGAGAACTTTCCCTTCAACGACTGTGTGGACA
AGATGGTGATCTGGTGGGAGGAGGGGAAGATGACCGCCAAGGTCGTGGAGTCGGCCAAAGC
CATTCTCGGAGGAAGCAAGGTGCGCGTGGACCAGAAATGCAAGTCCTCGGCCCAGATAGACC
CGACTCCCGTGATCGTCACCTCCAACACCAACATGTGCGCCGTGATTGACGGGAACTCAACG
ACCTTCGAACACCAGCAGCCGTTGCAAGACCGGATGTTCAAATTTGAACTCACCCGCCGTCT
GGATCATGACTTTGGGAAGGTCACCAAGCAGGAAGTCAAAGACTTTTTCCGGTGGGCAAAGG
ATCACGTGGTTGAGGTGGAGCATGAATTCTACGTCAAAAAGGGTGGAGCCAAGAAAAGACCC
GCCCCCAGTGACGCAGATATAAGTGAGCCCAAACGGGTGCGCGAGTCAGTTGCGCAGCCATC
GACGTCAGACGCGGAAGCATCGATCAACTACGCAGACAGGTACCAAAACAAATGTTCTCGTC

ACGTGGGCATGAATCTGATGCTGTTTCCCTGCAGACAATGCGAGAGAATGAATCAGAATTCAA
ATATCTGCTTCACTCACGGACAGAAAGACTGTTTAGAGTGCTTTCCCGTGTCAGAATCTCAAC
CCGTTTCTGTCGTCAAAAAGGCGTATCAGAAACTGTGCTACATTCATCATATCATGGGAAAGG
TGCCAGACGCTTGCACTGCCTGCGATCTGGTCAATGTGGATTTGGATGACTGCATCTTTGAAC
AATAATCTAGAGCCTGCAGTCTCGACAAGCTTGTCGAGAAGTACTAGAGGATCATAATCAGCC
ATACCACATTTGTAGAGGTTTTACTTGCTTTAAAAAACCTCCCACACCTCCCCCTGAACCTGA
AACATAAAATGAATGCAATTGTTGTTGTTAACTTGTTTATTGCAGCTTATAATGGTTACAAATAA
AGCAATAGCATCACAAATTTCACAAATAAAGCATTTTTTTCACTGCATTCTAGTTGTGGTTTGT
CCAAACTCATCAATGTATCTTATCATGTCTGGATCTGATCACTGCTTGAGCCTAGGAGATCCGA
ACCAGATAAGTGAAATCTAGTTCCAAACTATTTTGTCATTTTTAATTTTCGTATTAGCTTACGAC
GCTACACCCAGTTCCCATCTATTTTGTCACTCTTCCCTAAATAATCCTTAAAAACTCCATTTCCA
CCCCTCCCAGTTCCCAACTATTTTGTCCGCCCACAGCGGGGCATTTTTCTTCCTGTTATGTTTTT
AATCAAACATCCTGCCAACTCCATGTGACAAACCGTCATCTTCGGCTACTTT

In some embodiments, the AAV elements are derived from AAV of different serotypes, such as AAV1, AAV2, AAV3, AAV4, AAV5, AAV6, AAV7, AAV8, AAV9, AAV DJ, AAV DJ/8, AAV rh10, AAV Retro , AAV PHP.eB, AAV PHP.B, or AAV PHP.S. The capsid proteins of different serotypes of AAV recognize different receptors on the cell surface, so the infection efficiency to different tissue cells may vary greatly, showing certain organ-targeting specificity. The appropriate serotype of AAV can be selected according to the specific experimental purpose, so as to achieve specific high-efficiency transduction of a specific serotype of AAV to a specific type of cell and tissue.

In some embodiments, the AAV elements are derived from AAV1. In some embodiments, the AAV elements are derived from AAV2. In some embodiments, the AAV elements are derived from AAV3. In some embodiments, the AAV elements are derived from AAV4. In some embodiments, the AAV elements are derived from AAV5. In some embodiments, the AAV elements are derived from AAV6. In some embodiments, the AAV elements are derived from AAV7. In some embodiments, the AAV elements are derived from AAV8. In some embodiments, the AAV elements are derived from AAV9. In some embodiments, the AAV elements are derived from AAV DJ. In some embodiments, the AAV elements are derived from AAV DJ/8. In some embodiments, the AAV elements are derived from AAV rh10. In some embodiments, the AAV elements are derived from AAV Retro. In some embodiments, the AAV elements are derived from AAV PHP.eB/PHP.B/PHP.S.

The Cap proteins are structural proteins, usually including VP1, VP2, and VP3 structural proteins. It is believed that in some embodiments, three structural proteins can be assembled into the capsid protein of AAV.

The Rep proteins are non-structural proteins, usually including Rep78 and 52 proteins. It is believed that in some embodiments, Rep proteins regulate the replication and packaging of DNA.

In one embodiment, the AAV cis-acting elements are selected from inverted terminal repeat sequences (ITR sequences). It is believed that in some embodiments, the ITR sequences fold into a hairpin structure and are the only known cis-acting elements required for replication initiation of AAV DNA and packaging of recombinant AAV virions.

In some embodiments, the nucleic acid construct further includes an exogenous gene of interest. In some embodiments, the exogenous target gene is carried by AAV elements, that is, the exogenous target gene is embedded in between the AAV elements.

Specifically, in some embodiments, the exogenous target gene is embedded between two ITR sequences.

Preferably, in some embodiments, the size of the exogenous target gene is ≤3.5 kb. It is believed that in some embodiments, due to the size limitation between the two ITR sequences, the size of the exogenous target gene should generally not be too large, otherwise it may lead to poor packaging effect.

The type of the exogenous gene varies according to the purpose of the experiment. The exogenous gene can be a gene related to the treatment of various diseases, such as AADC, FVIII, FIX, and can also be a tool gene commonly used in laboratories, such as EGFP, mCherry gene, and the like.

It is believed that in some embodiments, the nucleic acid construct also includes a promoter for the IE protein-coding gene. It is believed that in some embodiments, the promoter of the IE protein-coding gene is selected from one or more of Gp64, pH, p6.9, and p10. In some embodiments, the promoter of the IE protein-coding gene includes Gp64. In some embodiments, the promoter of the IE protein-coding gene comprises pH. In some embodiments, the promoter of the IE protein-coding gene includes p6.9. In some embodiments, the promoter of the IE protein-coding gene includes p10.

Preferably, in some embodiments, the promoter of the IE protein-coding gene is selected from strong pH promoters. It is believed that in some embodiments, incorporation of an additional copy of ie gene into the nucleic acid construct and expressed under the control of a strong promoter pH above the endogenous IE levels of the virus increases the survival of cells at late stage following baculovirus infection .

In some embodiments, the IE protein is selected from proteins encoded by one or more (e.g., two or three) genes of Acie0, Acie01, and Acie02. In some embodiments, the IE protein is selected from a protein encoded by Acie0, i.e., IE0. The nucleotide sequence of Acie0 is shown in SEQ ID NO. 10, and the amino acid sequence of the IE0 protein is shown in SEQ ID NO. 11. In some embodiments, the said IE protein is selected from the protein encoded by Acie01, i.e., IE1. The nucleotide sequence of Acie01 is shown in SEQ ID NO.12, and the amino acid sequence of IE1 protein is shown in SEQ ID NO.13. In some embodiments, the IE protein is selected from the protein encoded by Acie02, namely IE2 protein. The nucleotide sequence of Acie02 is shown in SEQ ID NO.14, and the amino acid sequence of IE2 protein is shown in SEQ ID NO. 15.

The sequences of SEQ ID NO. 10-15 are as follows:
SEQ ID NO. 10: 
ATGATAAGAACCAGCAGTCACGTGCTGAACGTCCAGGAAAATATAATGACGTCAAACTGTGC
GTCATCGCCATATTCGTGCGAGGCAACGTCCGCTTGCGCAGAAGCTCAGCAGGTAATGATCGA
TAACTTTGTTTTCTTTCACATGTACAACGCCGACATACAAATTGACGCAAAGCTGCAATGCGG
CGTGCGCTCGGCCGCGTTTGCAATGATCGACGATAAACATTTGGAAATGTACAAGCATAGAAT
AGAGAATAAATTTTTTTATTACTATGATCAATGTGCCGACATTGCCAAACCCGACCGTCTGCCC
GATGACGACGGCGCGTGCTGTCACCATTTTATTTTTGATGCCCAACGTATTATTCAATGTATTAA
AGAGATTGAAAGCGCGTACGGCGTGCGTGATCGCGGCAATGTAATAGTGTTTTATCCGTACTT
GAAACAGTTGCGAGACGCGTTGAAGCTAATTAAAAACTCTTTTGCGTGTTGTTTTAAAATTAT
AAATTCTATGCAAATGTACGTGAACGAGTTAATATCAAATTGCCTGTTGTTTATTGAAAAGCTG
GAAACTATTAATAAAACTGTTAAAGTTATGAATTTGTTTGTAGACAATTTGGTTTTGTACGAAT
GCAATGTTTGTAAAGAAATATCTACGGATGAAAGATTTTTAAAGCCAAAAGAATGTTGCGAAT
ACGCTATATGCAACGCGTGCTGCGTTAACATGTGGAAGACGGCCACCACGCACGCAAAATGT
CCAGCGTGCAGGACATCGTATAAATAA
SEQ ID NO.11: 
MIRTSSHVLNVQENIMTSNCASSPYSCEATSACAEAQQVMIDNFVFFHMYNADIQIDAKLQCGVR
SAAFAMIDDKHLEMYKHRIENKFFYYYDQCADIAKPDRLPDDDGACCHHFIFDAQRIIQCIKEIES
AYGVRDRGNVIVFYPYLKQLRDALKLIKNSFACCFKIINSMQMYVNELISNCLLFIEKLETINKTV
KVMNLFVDNLVLYECNVCKEISTDERFLKPKECCEYAICNACCVNMWKTATTHAKCPACRTSYK
SEQ ID NO. 12: 
ATGATCCGTACATCCAGCCACGTCCTGAACGTCCAAGAAAACATCATGACTTCCAACTGTGCT
TCCAGCCCCTACTCCTGTGAGGCCACTTCAGCCTGCGCTGAGGCCCAGCAACTGCAGGTGGA
CACAGGTGGCGATAAGATCGTGAACAACCAGGTCACCATGACTCAAATCAACTTCAACGCTT
CCTACACCTCTGCCAGCACTCCCTCTCGTGCTAGCTTCGACAACTCATACTCGGAGTTCTGCG
ACAAGCAACCTAACGATTACTTGTCTTACTACAACCACCCAACCCCGGACGGAGCTGATACTG
TCATCTCCGACTCTGAAACCGCTGCCGCTAGCAACTTCCTCGCCTCAGTTAACTCGCTCACTG
 
ACAACGATTTGGTGGAGTGTCTGCTCAAGACCACTGACAACCTGGAGGAAGCTGTGTCCTCT
GCCTACTACAGCGAGTCACTCGAACAGCCAGTGGTCGAACAACCCTCTCCTAGCTCAGCTTA
CCACGCCGAGTCCTTCGAACACTCTGCTGGTGTCAACCAGCCGTCGGCCACAGGCACCAAGA
GGAAGTTGGACGAGTACCTGGATAACTCCCAGGGAGTTGTGGGTCAATTCAACAAGATCAAG
TTGAGACCTAAGTACAAGAAGAGCACCATCCAGTCATGCGCTACACTGGAACAAACCATCAA
CCACAACACTAACATCTGTACAGTGGCTTCCACCCAGGAGATCACTCACTACTTCACAAACGA
CTTCGCCCCCTACCTGATGAGGTTCGACGATAACGACTACAACTCGAACAGATTCTCCGATCA
CATGTCTGAAACCGGTTACTACATGTTCGTCGTTAAGAAGTCCGAGGTGAAGCCTTTCGAAAT
CATCTTCGCCAAGTACGTCTCTAACGTGGTCTACGAGTACACAAACAACTACTACATGGTTGA
CAACCGTGTGTTCGTTGTGACCTTCGATAAGATCCGCTTCATGATCAGCTACAACCTGGTTAAG
GAGACTGGCATCGAAATCCCACACTCACAGGACGTCTGCAACGATGAGACCGCCGCTCAAAA
CTGCAAGAAGTGTCACTTCGTGGACGTCCACCACACATTCAAGGCCGCTCTGACCTCCTACTT
CAACCTCGATATGTACTACGCTCAGACAACCTTCGTGACCTTGCTGCAATCACTCGGCGAGCG
TAAGTGTGGATTCCTCTTGTCGAAGTTGTACGAGATGTACCAGGACAAGAACCTCTTCACTTT
GCCCATCATGCTGAGCCGCAAGGAATCAAACGAGATCGAAACCGCCTCTAACAACTTCTTCGT
CTCGCCATACGTTTCCCAGATCCTCAAGTACTCGGAGTCCGTCCAATTCCCGGACAACCCTCC
CAACAAGTACGTCGTTGATAACCTGAACCTCATCGTGAACAAGAAGAGCACTCTGACATACA
AGTACTCGTCCGTCGCTAACCTGCTCTTCAACAACTACAAGTACCACGACAACATCGCTTCTA
ACAACAACGCCGAGAACCTCAAGAAGGTCAAGAAGGAAGACGGAAGCATGCACATCGTTGA
GCAGTACTTGACTCAAAACGTCGATAACGTTAAGGGTCACAACTTCATCGTGTTGTCCTTCAA
GAACGAGGAAAGGCTGACCATCGCTAAGAAGAACAAGGAGTTCTACTGGATCTCTGGCGAAA
TCAAGGACGTTGATGTGAGCCAGGTCATCCAAAAGTACAACAGATTCAAGCACCACATGTTC
GTGATCGGCAAGGTCAACCGTCGCGAGTCAACTACACTGCACAACAACTTGCTGAAGCTCTT
GGCCTTGATCCTGCAGGGACTGGTGCCACTCTCCGACGCCATCACATTCGCCGAGCAAAAGC
TCAACTGCAAGTACAAGAAGTTCGAGTTCAACTAA
SEQ ID NO.13 : 
MIRTSSHVLNVQENIMTSNCASSPYSCEATSACAEAQQLQVDTGGDKIVNNQVTMTQINFNASYT
SASTPSRASFDNSYSEFCDKQPNDYLSYYNHPTPDGADTVISDSETAAASNFLASVNSLTDNDLVE
CLLKTTDNLEEAVSSAYYSESLEQPVVEQPSPSSAYHAESFEHSAGVNQPSATGTKRKLDEYLDNS
QGVVGQFNKIKLRPKYKKSTIQSCATLEQTINHNTNICTVASTQEITHYFTNDFAPYLMRFDDNDY
NSNRFSDHMSETGYYMFVVKKSEVKPFEIIFAKYVSNVVYEYTNNYYMVDNRVFVVTFDKIRFM
ISYNLVKETGIEIPHSQDVCNDETAAQNCKKCHFVDVHHTFKAALTSYFNLDMYYAQTTFVTLLQ
SLGERKCGFLLSKLYEMYQDKNLFTLPIMLSRKESNEIETASNNFFVSPYVSQILKYSESVQFPDNP
 
PNKYVVDNLNLIVNKKSTLTYKYSSVANLLFNNYKYHDNIASNNNAENLKKVKKEDGSMHIVE
QYLTQNVDNVKGHNFIVLSFKNEERLTIAKKNKEFYWISGEIKDVDVSQVIQKYNRFKHHMFVIG
KVNRRESTTLHNNLLKLLALILQGLVPLSDAITFAEQKLNCKYKKFEFN
SEQ ID NO. 14: 
ATGAGTCGCCAAATCAACGCCGCCACTCCCAGCAGCCGCCGCCACAGGCTGTCTCTCAG
CCGTCGCCGCATCAACTTTACAACATCTCCCGAAGCCCAGCCGTCTTCAAGCAGTCGCAGCCA
GCCGTCTTCAAGCAGTCGCAGCCATCGCCGTCAGGAGCGGCGTCAGGAGCAGCGTGTCAGC
GAAGAAAACGTGCAGATTATCGGGAACGTCAACGAGCCGTTGACGCGCACCTACCATCGTCA
GGGTGTCACGTATTACGTGCACGGTCAGGTTAACATTAGCAATGACGATCCGCTATTAAGTCA
AGAGGATGACGTCATACTAATTAATAGTGAAAATGTGGATCGTGAACGGTTTCCCGACATCAC
TGCCCAGCAGTACCAGGATAACATTGCGTCGGAGACAGCTGCGCAGAGGGCTCTGCAACGAG
GTTTAGATCTTGAGGCTCAGCTGATGAATGAGATTGCCCCAAGGTCTCCCACTTATAGTCCATC
TTATTCGCCGAATTACGTAATACCACAGTCGCCAGATTTGTTTGCCTCGCCGCAGTCTCCGCAG
CCGCAGCAGCAGCAGCAGCAGCAATCAGAACCCGAAGAAGTAGAGGTTTCGTGTAATAT
TTGTTTTACTACTTTTAAAGACACTAAAAACGTAAATTCCTCGTTTGTGACTTCGATTCATTGTA
ACCATGCTGTGTGTTTCAAGTGTTATGTCAAGATAATTATGGACAATTCTGTGTACAAATGTTTT
TGCAGCGCTACTTCATCAGATTGTCGCGTGTACAATAAGCACGGGTATGTAGAATTTATGCCCA
TTAACGTCACTCGTAACCAGGATTCCATCAAACAGCATTGGCGCGAGCTTTTAGAAAATAACA
CGGTCAACAATCACACCACGGACTTGAACTATGTGGAGCAATTGCAAAAAGAACTGTCCGAG
CTGCGAGCCAAGACCAGCCAAGTTGAACATAAAATGACCATGTTAAACAGCGACTACATTATG
CTTAAACACAAGCATGCTGTCGCCGAATTAGATTTACAAAAGGCAAACTATGACTTGCAAGAA
TCTACCAAGAAATCAGAAGAGTTGCAATCGACTGTGAATAATCTGCAAGAACAATTGCGTAAG
CAGGTGGCCGAGTCTCAAGCCAAATTTTCAGAGTTTGAGCGCAGTAACTCTGATTTAGTTTCT
AAGTTACAAACTGTTATGTCTAGACGTTAA
SEQ ID NO.15:

MSRQINAATPSSSRRHRLSLSRRRINFTTSPEAQPSSSSRSQPSSSSRSHRRQERRQEQRVSEENVQI
IGNVNEPLTRTYHRQGVTYYVHGQVNISNDDPLLSQEDDVILINSENVDRERFPDITAQQYQDNI
ASETAAQRALQRGLDLEAQLMNEIAPRSPTYSPSYSPNYVIPQSPDLFASPQSPQPQQQQQQSEP
EEEVEVSCNICFTTFKDTKNVNSSFVTSIHCNHAVCFKCYVKIIMDNSVYKCFCSATSSDCRVYN
KHGYVEFMPINVTRNQDSIKQHWRELLENNTVNNHTTDLNYVEQLQKELSELRAKTSQVEHKM
TMLNSDYIMLKHKHAVAELDLQKANYDLQESTKKSEELQSTVNNLQEQLRKQVAESQAKFSEF
ERSNSDLVSKLQTVMSRR

It is believed that in some embodiments, the IE1 protein is a product of the immediate early gene 1 (ie1) of baculovirus, and the IE protein may also be referred to as a transcriptional regulator protein of baculovirus. It is a multifunctional protein with the ability to activate early and late viral genes and participate in viral genome replication, and participates in the regulation of the viral cycle.

In some embodiments, the recombination homologous region of baculovirus (hr) is selected from one or more (e.g., two, three, four or five) of hr1 (its nucleotide sequence shown in SEQ ID NO.16), hr2 (its nucleotide sequence shown in SEQ ID NO.17 ), hr3 (its nucleotide sequence is shown in SEQ ID NO.18), hr4 (the nucleotide sequence of hr4 left is shown in SEQ ID NO. 19, and the nucleotide sequence of hr4 right is shown in SEQ ID NO.20), and hr5 (its nucleotide sequence is shown in SEQ ID NO.21).

The sequences of SEQ ID NO. 16-21 are as follows:
SEQ ID NO. 16: 
ATCGATGTTGACCCCAACAAAAGATTTATAATTAATCATAATCACGAACAACAACAAGTCAATG
AAACAAATAAACAAGTTGTCGATAAAACATTCATAAATGACACAGCAACATACAATTCTTGCA
TAATAAAAATTTAAATGACATCATATTTGAGAATAACAAATGACATTATCCCTCGATTGTGTTTT
ACAAGTAGAATTCTACCCGTAAAGCGAGTTTAGTTTTGAAAAACAAATGACATCATTTGTATAA
TGACATCATCCCCTGATTGTGTTTTACAAGTAGAATTCTATCCGTAAAGCGAGTTCAGTTTTGA
AAACAAATGAGTCATACCTAAACACGTTAATAATCTTCTGATATCAGCTTATGACTCAAGTTAT
GAGCCGTGTGCAAAACATGAGATAAGTTTATGACATCATCCACTGATCGTGCGTTACAAGTAG
AATTCTACTCGTAAAGCCAGTTCGGTTATGAGCCGTGTGCAAAACATGACATCAGCTTATGAC
TCATACTTGATTGTGTTTTACGCGTAGAATTCTACTCGTAAAGCGAGTTCGGTTATGAGCCGTG
TGCAAAACATGACATCAGCTTATGAGTCATAATTAATCGTGCGTTACAAGTAGAATTCTACTCG
TAAAGCGAGTTGAAGGATCATATTTAGTTGCGTTTATGAGATAAGATTGAAAGCACGTGTAAA
ATGTTTCCCGCGCGTTGGCACAACTATTTACAATGCGGCCAAGTTATAAAAGATTCTAATCTGA
TATGTTTTAAAACACCTTTGCGGCCCGAGTTGTTTGCGTACGTGACTAGCGAAGAAGATGTGT
GGACCGCAGAACAGATAGTAAAACAAAACCCTAGTATTGGAGCAATAATCGAT
SEQ ID NO. 17: 
TGAGCAAAACACAACCGGCAAATTCTCGGCGGCCGTTTGGGAATGCGGAATAATTGCCATATG
TAAATGATGTCATCGGTTCTAACTCGCTTTACGAGTAGAATTCTACGTGTAAAACATAATCAAG
AGATGATGTCATTTGTTTTCAAAACTGAACTCAAGAAATGATGTCATTTGTTTTCAAAACTG
AACTGGCTTTACGAGTAGAATTCTACTTGTAAAACACAATCGAGAGATGATGTCATATTTGCA
CACGGCTCTAATTAAACTCGCTTTACGAGTAAAATTCTACTTGTAACGCATGATCAAGGGATGA
TGTCATTGGATGAGTCATTTGTTTTCAAAACTAAACTCGCTTTACGAGTAGAATTCTACTTGT
 
AAAACACAATCAAGGGATGATGTCATTATACAAATGATGTCATTTGTTTTTCAAAACTAAACTC
GCTTTACGGGTAGAATTCTACTTGTAAAACAGCAACTCGAGGGATGATGTCATCCTTTACTCGA
TGATTATAAACGTGTTTATGTATGACTCATTTGTTTTTCAAAACTAAACTCGCTTTACGAGTAGA
ATTCTACTTGTAACGCACGATCAAGGGATGATGTCATTTATTTGTGCAAAGCTCGATGTCATCT
TTTGCACACGATTATAAACACAATCCAAATAATGACTCATTTGTTTTCAAAACTGAACTCGCTT
TACGAGTAGAATTCTACTTGTAAAACACAATCAAGGGATGATGTCATTTTCAAAATGATGTCAT
TTGTTTTTCAAAACTAAACTCGCTTTACGAGTAGAATTCTACTTGTAAAACACAATCAAGGGAT
GATGTCATTTTAAAAATGATCATTTGTTTTTCAAAACTAAACTCGCTTTACGAGTAGAATTCTAC
GTGTAAAACACAATCAAGGGATGATGTCATTTACTAAATAAAATAATTATTTAAATAAAACTGT
TTTTTATTGTCAAATACACATTGATTCAC
SEQ ID NO.18: 
ACGCGTAGAATTCTACTTGTAAAGCAAGTTAAAATAAGCCGTGTGCAAAAATGACATCAGACA
AATGACATCATCTACCTATCATGATCATGTTAATAATCATGTTTTAAAATGACATCAGCTTATGAC
TAATAATTGATCGTGCGTTACAAGTAGAATTCTACTCGTAAAGCGAGTTTAGTTTTGAAAACAA
ATGAGTCATCATTAAACATGTTAATAATCGTGTATAAAGGATGACATCATCCACTAATCGTGCGT
TACAAGTAGAATTCTACTCGTAAAGCGAGTTCGGTTTTGAAAAACAAATGACATCATTTCTTG
ATTGTGTTTTACACGTAGAATTCTACTCGTAAAGTATGTTCAGTTTAAAAAACAAATGACATCA
TTTTACAGATGACATCATTTCTTGATTATGTTTTACAAGTAGAATTCTACTCGTAAAGCGAGTTT
AGTTTTAAAAAACAAATGACATCATCTCTTGATTATGTTTTACAAGTAGAATTCTACTCGTAAA
GCGAGTTTAGTTTTGAAAAACAAATGACATCATCTCTTGATTATGTTTTACAAGTAGAATTCTA
CTCGTAAAGCGAGTTTAGTTTTGAAAAACAAATGACATCATCCCTTGATCATGCGTTACAAGT
AGAATTCTACTCGTAAAGCGAGTTGAATTTTGATTACAATATT
SEQ ID NO. 19: 
ATGCATATAATTGTGTACAAAATATGACTCATTAATCGATCGTGCGTTACAAGTAGAATTCTACT
GGTAAAGCAAGTTCGGTTGTGAGCCGTGTGCAAAACATGACATCATAACTAATCATGTTTATAA
TCATGTGCAAAATATGACATCATCCGACGATTGTGTTTTACAAGTAGAATTCTACTCGTAAAGC
GAGTTTAAAAATTTTGTGACGTCAATGAAACAACGTGTAATATTTTTTACAATATTTAAGTGAA
ACATTATGACTTCCAATAATTTTGTGGATGTGGATACGTTTGCAAGACAATTGATTACAGATAAA
TGTAGTGCTCTAATCGAAAGATGCGGATCTGTTGCCGGCAAACATTTTAGAGATTAGTAGAGA
AAGGCCAGAGACAAGTATTTTGAGGTGCCAACTCAAAAAAACTATGAATACATTAAAAAATTA
TTTTTACGAACAAAATATATGGACGATTCGATAGATTATAAAGATTTTAACAGACGCATCCTATT
GATAGTTTTTAAATTCGCTTTAAACAAGAGCACCAACTACTTTCCATCGTACTAAAGAGATCAT
 
CGAGGTGGCCATTAAACGTTTAAACAAAATTAACCCCGATTTAAAGAGTTCTCCGCGCAATGC
TTCAGCATTACAAATGAATGTTTGGAAAATCTAGA
SEQ ID NO.20: 
AACTGGCTTTACGAGTAGAATTCTACTTGTAAAACACAATCAAGAAATGATGTCATTTTTGTAC
GTGATTATAAACATGTTTAAACATGGTACATTGAACTTAATTTTTGCAAGTTGATAAACTAGATT
AATGTATGACTCATTTGTTTGTGCAAGTTGATAAACGTGATTAATATATGACTCATATGTTTGTG
CAAAAATGGTGTCATCGTACAAACTCGCTTTACGAGTAGAATTCTACTTGTAAAACACAATCG
AGGGATGATGTCATTTGTAGAATGATGTCATTTGTTTTTTCAAAACCGAACTCGCTTTACGAGT
AGAATTCTACTTGTAAAACACAATCGAGGGATGATGTCATTTGTAGAATGATGTCATCGTACAA
ACTCGCTTTACGAGTAGAATTCTAGTAAAACAC
SEQ ID NO.21:

TTGAAAATTTATTGCCTAATATTATTTTTGTCAGTTCGTTGTCATTTATTAATTTGGATGATGT
CCATTTGTTTTTAAAATTGAACTGGCTTTACGAGTAGAATTCTACGCGTAAAACACAATCAA
GTATGAGTCATAAGCTGATGTCATGTTTTGCACACGGCTCATAACCGAACTGGCTTTACGAG
TAGAATTCTACTTGTAACGCACGATCGAGTGGATGATGGTCATTTGTTTTTCAAATCGAGAT
GATGTCATGTTTTGCACACGGGCTCATAAACTGCTTTACGAGTAGAATTCTACGTGTAACGC
ACGATCGATTGATGAGTCATTTGTTTTGCAATATGATATCATACAATATGACTCATTTGTTTT
TCAAAACCGAACTTGATTTACGGGTAGAATTCTACTCGTAAAGCACAATCAAAAAGATGATG
TCATTTGTTTTTCAAAACTGAACTCTCGGCTTTACGAGTAGAATTCTACGTGTAAAACACAAT
CAAGAAATGATGTCATTTGTTATAAAAATAAAAGCTGATGTCATGTTTTGCACATGGCTCAT
AACTAAACTCGCTTTACGGGTAGAATTCTACGCGTAAAACATGATTGATAATTAAATAATTC
ATTTGCAAAGCTATACGTTAAATCAAACGGACGTTATGGAATTGTATAATATTAAATATGCA
ATTGATCCAACAAATAAAATTATAATAGAGCAAGTCGAC

Preferably, in some embodiments, the recombination homologous region of baculovirus is selected from hr1. It is believed that in some embodiments, hr1 is a repetitive sequence scattered throughout the baculovirus genome, and that hr1 is an origin of replication of baculovirus and also functions as an enhancer. Although the enhancement effect was not obvious in the early stage of infection, it became more significant in the late stage of infection. In some embodiments, the recombination homologous region of baculovirus (hr) includes hr2. In some embodiments, the recombination homologous region of baculovirus (hr) includes hr3. In some embodiments, the recombination homologous region of baculovirus (hr) includes hr4. In some embodiments, the recombination homologous region of baculovirus (hr) includes hr5.

In some embodiments, the nucleic acid construct further includes a baculovirus promoter. In some embodiments, the baculovirus promoter is selected from one or more (e.g., two, three, or four) of polyhedron promoter (pH), Gp64, p6.9, and p10. It is believed that in some embodiments, hr is connected in *cis* to the baculovirus promoter to promote IE-mediated transactivation, and at the same time, IE will bind to hr1 in a dimer form to increase the expression of downstream proteins.

In some embodiments, the structure of the nucleic acid construct includes: IE gene expression cassette-Cap gene expression cassette-hr 1- ITR -exogenous target gene expression cassette-ITR-Rep gene expression cassette. In some embodiments, the structure of the nucleic acid construct is: IE gene expression cassette-Cap gene expression cassette-ITR-exogenous target gene expression cassette-ITR-Rep gene expression cassette.

The gene expression cassette includes a gene and its promoter.

In a preferred embodiment, the structure of the nucleic acid construct includes: pA-IE1-pH-pA-Cap-p6.9p10-hr1-ITR-exogenous target gene and its promoter-pA-ITR-pH-Rep-pA. In a preferred embodiment, the structure of the nucleic acid construct is: pA-IE1-pH-pA-Cap-p6.9p10-hr1-ITR-exogenous target gene and its promoter-pA-ITR-pH-Rep-pA.

Specifically, in some embodiments, the nucleic acid construct is an AAV vector or a recombinant baculovirus vector. In some embodiments, the nucleic acid construct is an AAV vector. In some embodiments, the nucleic acid construct is a recombinant baculovirus vector. In some embodiments, the recombinant baculovirus vector is a recombinant baculovirus shuttle vector.

In some embodiments, the AAV vector or recombinant baculovirus vector further includes a vector backbone.

In some embodiments, the vector backbone can be selected from suitable vector backbones sold in the market, such as pFastBacdual, pFastBac1, pFastBacHTA, pFastBacHTB, pFastBacHTC, or the like.

In one embodiment, the vector backbone is pFastBacdual.

In some embodiments, the rAAV titer obtained by packaging the nucleic acid construct is at least 1×10¹² VG/mL (for example, at least 1.5×10¹², 2×10¹², 2.5×10¹², 3×10¹², 3.5x 10¹², 4×10¹², 4.5×10¹², 5×10¹², 5.5×10¹², 6×10¹², 7×10¹², 7.5×10¹², 8×10¹², 8.5×10¹², 9×10¹², 9.5×10¹², 1×10¹³, 5×10¹³, or 1×10¹⁴ VG/mL), that is, the copy number of AAV genome contained in each mL of virus culture medium is at least 1×10¹² (for example, at least 1.5×10¹², 2×10¹², 2.5×10¹², 3×10¹², 3.5×10¹², 4×10¹², 4.5×10¹², 5×10¹², 5.5×10¹², 6×10¹², 7×10¹², 7.5×10¹², 8×10¹², 8.5×10¹², 9×10¹², 9.5×10¹², 1×10¹³, 5×10¹³, or 1×10¹⁴).

In some embodiments, the rAAV titer obtained by packaging the nucleic acid construct is at least 2.60E+12 VG/mL, that is, the copy number of the AAV genome contained in each mL of virus culture medium is at least 2.60E+12.

The yield of the rAAV is determined by qPCR method, and the detection steps are as follows:
The standard used in quantitative PCR is pAAV-MCS plasmid linearized by single restriction enzyme digestion with PvuI-HF (NEB).

The primer sequences used in quantitative PCR are:
ITR Forward primer 5'-GGAACCCCTAGTGATGGAGTT-3' (SEQ ID NO: 2)
ITR Reverse primer 5'-CGGCCTCAGTGAGCGA-3' (SEQ ID NO: 3)

The running program of the quantitative PCR was: 95°C for 60 s, (95°C for 15 s, 60°C for 30 s, 40 cycles). After plotting the standard curve according to the CT value obtained by quantitative PCR and the concentration of the standard, the titer of the sample was calculated.

The second aspect of the present invention provides a recombinant baculovirus, which is obtained by constructing any one of the nucleic acid constructs through the baculovirus system, or is obtained by constructing nucleic acid constructs by including any element of the said nucleic acid constructs through the baculovirus system.

The nucleic acid constructs comprising any element from any of said nucleic acid constructs mainly refer to: nucleic acid constructs comprising AAV elements, nucleic acid constructs comprising a polynucleotide encoding an IE protein, nucleic acid constructs comprising polynucleotides encoding recombination homologous region of baculovirus. The nucleic acid constructs comprising AAV elements can also be selected from nucleic acid constructs comprising a polynucleotide encoding Cap proteins, nucleic acid constructs comprising a polynucleotide encoding Rep proteins, and nucleic acid constructs comprising AAV cis-acting elements. The recombinant baculovirus can be constructed from the above nucleic acid constructs together through a baculovirus system.

The baculovirus system is selected from Bac-to-Bac system (derived from ThermoFisher/Invitrogen), flashBac/BacMagic system (derived from Mirus/EMD/OET/Nextgen), BaculoDirect system (derived from ThermoFisher/Invitrogen), and BacPAK6/Baculogold system (derived from BD Biosciences/Clonetech).

Specifically, the recombinant baculovirus is obtained by transforming competent cells with the nucleic acid construct, extracting bacmid, and transfecting Sf9 insect cells.

The competent cells can be selected from any applicable competent cells in the art, as long as the purpose of the present invention is not limited. For example, the competent cells can be DH10Bac.

The third aspect of the present invention provides an adeno-associated virus (AAV), which is obtained by packaging any one of the recombinant baculoviruses. The adeno-associated virus can be used to treat various diseases, such as hemophilia, spinal muscular atrophy, Duchenne muscular dystrophy, Parkinson's disease, age-related macular degeneration, and the like.

The fourth aspect of the present invention provides a cell line, which is a cell line infected by any one of the recombinant baculoviruses.

In some embodiments, the cell line is an insect cell line, such as Sf9 cells, Sf21 cells, High5 cells.

The fifth aspect of the present invention provides an AAV vector system, which includes a baculovirus system and the nucleic acid construct.

The sixth aspect of the present invention provides the construction method of the nucleic acid construct, the construction method comprising integrating the AAV elements carrying the exogenous gene of interest and the polynucleotide encoding the IE protein into the backbone of the baculovirus vector.

The construction method also includes integrating the polynucleotide encoding the recombination homologous region of baculovirus into the backbone of the baculovirus vector.

In some embodiments, the AAV elements include a polynucleotide encoding Cap proteins, a polynucleotide encoding Rep proteins, and AAV cis-acting elements.

In some embodiments, the AAV cis-acting elements are selected from inverted terminal repeat sequences (ITR sequences).

Specifically, in some embodiments, the polynucleotide encoding the IE protein is linked to a strong promoter pH.

In some embodiments, the polynucleotide encoding the recombination homologous region of baculovirus is linked in *cis* to a baculovirus promoter. In some embodiments, the baculovirus promoter is p6.9 or p 10.

In some embodiments, the backbone of the baculovirus vector is selected from pFastBacdual, pFastBac1, pFastBacHTA, pFastBacHTB, pFastBacHTC, and etc.

In some embodiments, the recombination homologous region of baculovirus is selected from one or more (e.g., two, three, four, or five) of hr1, hr2, hr3, hr4, and hr5.

The seventh aspect of the present invention provides an AAV production method, the production method comprising the following steps: infecting insect cell lines with the recombinant baculovirus.

The production method can improve the yield of the AAV.

In some embodiments, the cell line is selected from an insect cell line. In one embodiment, the insect cell line is selected from Sf9 cells.

The eighth aspect of the present invention provides a method of treating diseases, said method comprising administering an effective amount of any one of the AAVs to patients.

In some embodiments, the diseases include, for example, hemophilia, spinal muscular atrophy, Duchenne muscular dystrophy, Parkinson's disease, age-related macular degeneration, and the like.

The invention also includes any of the following numbered paragraphs:
1, A nucleic acid construct, characterized in that, the nucleic acid construct comprises: AAV elements, the polynucleotide encoding IE protein, and the AAV elements comprise the polynucleotide encoding Cap proteins, the polynucleotide encoding Rep proteins, and AAV cis-acting elements.
2. The nucleic acid construct according to paragraph 1, wherein the nucleic acid construct further comprises a polynucleotide encoding a recombination homologous region of baculovirus.
3. The nucleic acid construct according to paragraph 2, wherein the IE protein is selected from one or more encoded proteins of Acie0, Acie01, and Acie02, and/or, the recombination homologous region of baculovirus is selected from one or more of hr1, hr2, hr3, hr4, and hr5.
4. Any one of the nucleic acid constructs described in paragraphs 1-3, characterized in that the nucleic acid construct also includes the promoter of the IE protein-coding gene, and the promoter of the IE protein-coding gene is selected from one or more of Gp64, pH, p6.9, and p 10.
5. Any one of the nucleic acid constructs described in paragraphs 1-4, wherein the nucleic acid construct further comprises a baculovirus promoter connected to a recombination homologous region of baculovirus, and the baculovirus promoter is preferably one or more of pH, Gp64, p6.9, and p 10.
6. Any one of the nucleic acid constructs described in paragraphs 1-5, wherein the AAV cis-acting elements are selected from ITR sequences.
7. Any one of the nucleic acid constructs described in paragraphs 1-6, wherein the nucleic acid construct further comprises an exogenous gene of interest embedded in between AAV elements.
8. Any one of the nucleic acid constructs described in paragraphs 1-7, wherein the structure of the nucleic acid construct is: IE gene expression cassette-Cap gene expression cassette-ITR-exogenous target gene expression cassette-ITR- Rep gene expression cassette.
9. Any one of the nucleic acid constructs described in paragraphs 1-8, wherein the nucleotide sequence of the nucleic acid construct is as SEQ ID NO. 1.
10. Any one of the nucleic acid constructs described in paragraphs 1-9, wherein the nucleic acid construct is an AAV vector or a recombinant baculovirus vector, and the recombinant baculovirus vector is preferably a recombinant baculovirus shuttle vector.
11. A recombinant baculovirus, characterized in that, the recombinant baculovirus is obtained by constructing the nucleic acid construct described in any one of paragraphs 1-10 through a baculovirus system, or by jointly constructing any of the nucleic acid constructs containing any element of the nucleic acid constructs described in paragraphs 1-10 through the baculovirus system.
12. An adeno-associated virus, characterized in that the adeno-associated virus is obtained after infection of cells with the recombinant baculovirus described in paragraph 11 and packaging.
13. A cell line, characterized in that the cell line is a cell line infected with the recombinant baculovirus described in paragraph 11.
14. An AAV vector system, characterized in that the AAV vector system comprises a baculovirus system and any one of the nucleic acid constructs described in paragraphs 1-10.
15. A method for constructing any one of the nucleic acid constructs described in paragraphs 1-10, characterized in that the construction method comprises integrating AAV elements carrying an exogenous gene of interest and the polynucleotide encoding an IE protein into the backbone of a baculovirus vector.
16. The construction method according to paragraph 15, wherein the construction method includes one or more of the following features:
   1) The AAV elements include a polynucleotide encoding Cap proteins, a polynucleotide encoding Rep proteins, and AAV cis-acting elements, and the AAV cis-acting elements are preferably ITR sequences;
   2) The IE protein is encoded by one or more of Acie0, Acie01, and Acie02 genes;
   3) The backbone of the baculovirus vector is selected from one of pFastBacdual, pFastBac1, pFastBacHTA, pFastBacHTB, and pFastBacHTC;
   4) The recombination homologous region of baculovirus is selected from one or more of hr1, hr2, hr3, hr4, and hr5;
   5) Integrating the polynucleotide encoding the recombination homologous region of baculovirus into the backbone of the baculovirus vector.
17. A production method of adeno-associated virus, characterized in that the production method comprises the following steps: infecting insect cell lines with the recombinant baculovirus described in paragraph 11.

Embodiments of the present invention are described below through specific examples, and those skilled in the art can easily understand other advantages and effects of the present invention from the content disclosed in this specification. The present invention can also be implemented or applied through other different specific implementation modes, and various modifications or changes can be made to the details in this specification based on different viewpoints and applications without departing from the spirit of the present invention.

Before further describing the specific embodiments of the present invention, it should be understood that the protection scope of the present invention is not limited to the following specific embodiments; it should also be understood that the terms used in the examples of the present invention are to describe specific embodiments, not intended to limit the protection scope of the present invention; in the description and claims of the present invention, unless the context clearly indicates otherwise, the singular forms "a", "an" and "the" include plural forms.

When the examples give numerical ranges, it should be understood that, unless otherwise stated in the present invention, the two endpoints of each numerical range and any value between the two endpoints can be selected. Unless defined otherwise, all technical and scientific terms used herein have the same meaning as commonly understood by one of ordinary skill in the art. In addition to the specific methods, equipment, and materials used in the embodiments, according to those skilled in the art's grasp of the prior art and the description of the present invention, any methods, equipment, and materials of the prior art similar or equivalent to the practice of the present invention can also be used.

The following examples take EGFP as an exogenous target gene as an example.

### Example 1. Construction of recombinant baculovirus shuttle vector and acquisition of recombinant baculovirus

Identify related genes: Both Cap and Rep protein-coding genes are derived from the AAV2 genome (GenBank: AF043303.1), according to the ribosome leak scanning mechanism reference (Smith RH, Levy JR, Kotin RM: A simplified baculovirus-AAV expression vector system coupled with one-step affinity purification yields high-titer rAAV stocks from insect cells. Mol Ther 2009, 17(11): 1888-1896) point mutations of bases were generated; Acie01 (GenBank: NC_001623.1), recombination homologous region of baculovirus hr1 (GenBank: M14313.1).

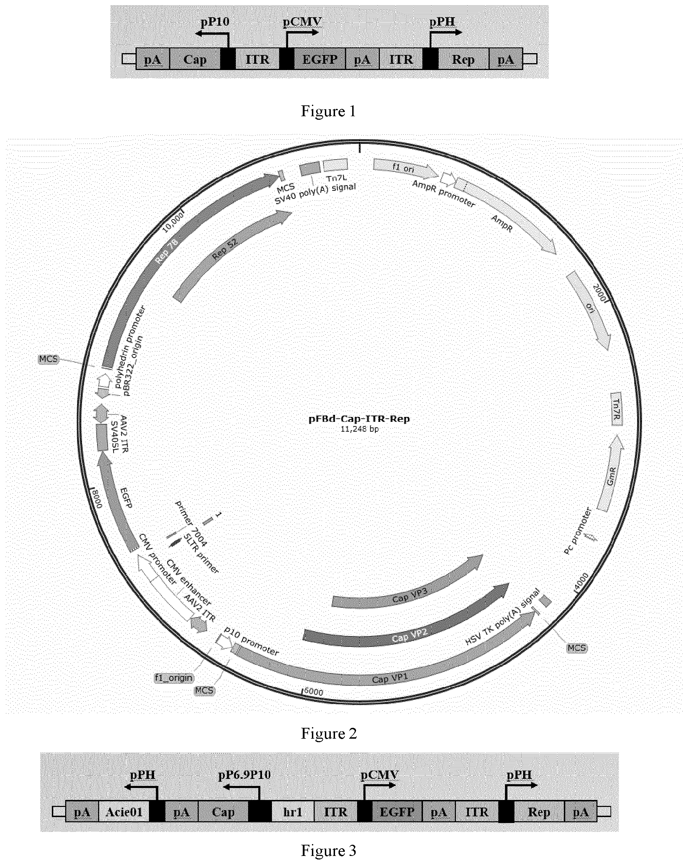

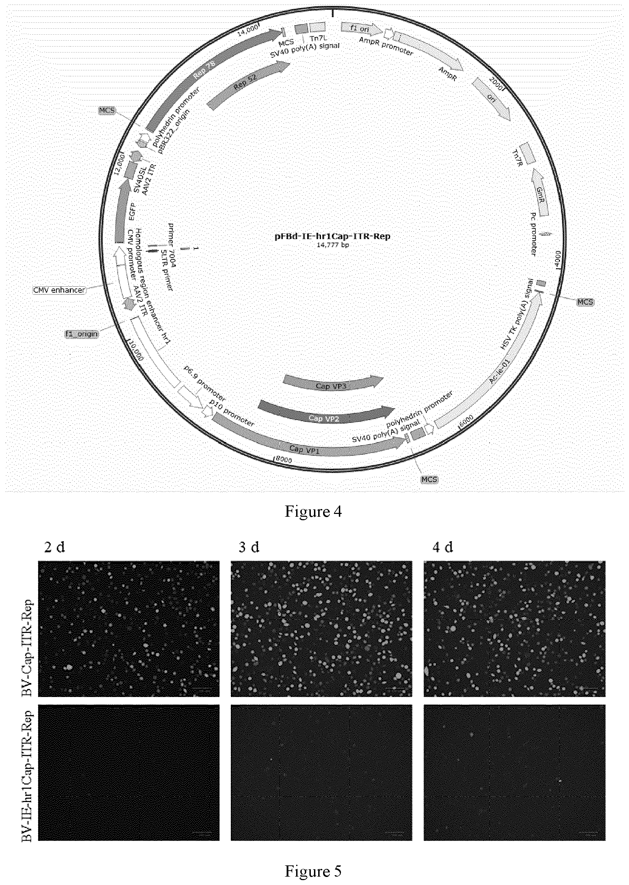

Entrust the gene company to synthesize the corresponding sequences: Cap gene sequence (nucleotide sequence SEQ ID NO: 4), Rep gene sequence (nucleotide sequence SEQ ID NO: 5), hr1 and p6.9, p10 promoter combination (hr1p6.9p10, nucleotide sequence of SEQ ID NO: 6), pH promoter and Acie01 combination (pH-Acie01, nucleotide sequence of SEQ ID NO: 7). The above sequences and the ITR-CMV-EGFP sequence in the pAAV-EGFP vector (modified by Kanglin Biotech (Hangzhou) Co., Ltd.) were cloned into pFastBacdual (Invitrogen) using the homologous recombination method well known in the art, and two recombinant baculovirus shuttle vectors pFBd-Cap-ITR-Rep (Figure 1 and Figure 2, nucleotide sequence SEQ ID NO: 8) and pFBd-IE-hr I Cap -ITR-Rep (Figure 3 and Figure 4, nucleotide sequence SEQ ID NO: 9 ) were obtained after identification by sequencing.

The sequences of SEQ ID NO.4-9 are as follows:
SEQ ID NO: 4: 
ACGGCTGCCGACGGTTATCTACCCGATTGGCTCGAGGACACTCTCTCTGAAGGAATAAGACAG
TGGTGGAAGCTCAAACCCGGCCCACCACCACCAAAGCCCGCAGAGCGGCATAAGGACGACA
GCAGGGGCCTTGTGCTTCCCGGGTACAAGTACCTCGGACCCTTCAACGGACTGGACAAGGGA
GAGCCGGTGAACGAGGCAGACGCCGCTGCCCTCGAGCACGACAAAGCCTACGACCGGCAGC
TCGACAGCGGAGACAACCCTTACCTCAAGTACAACCACGCCGACGCGGAGTTCCAGGAGCG
CCTTAAAGAAGATACGTCTTTTGGGGGCAACCTCGGACGAGCAGTCTTCCAGGCGAAAAAGA
GGGTTCTTGAACCTCTGGGCCTGGTTGAGGAACCTGTTAAGACGGCTCCGGGAAAAAAGAG
GCCGGTAGAGCACTCTCCTGTGGAGCCAGACTCCTCCTCGGGAACCGGAAAGGCGGGCCAG
CAGCCTGCAAGAAAAAGATTGAATTTTGGTCAGACTGGAGACGCAGACTCAGTACCTGACCC
CCAGCCTCTCGGACAGCCACCAGCAGCCCCCTCTGGTCTGGGAACTAATACGATGGCTACAG
GCAGTGGCGCACCAATGGCAGACAATAACGAGGGCGCCGACGGAGTGGGTAATTCCTCGGG
AAATTGGCATTGCGATTCCACATGGATGGGCGACAGAGTCATCACCACCAGCACCCGAACCT
GGGCCCTGCCCACCTACAACAACCACCTCTACAAACAAATTTCCAGCCAATCAGGAGCCTCG
AACGACAATCACTACTTTGGCTACAGCACCCCTTGGGGGTATTTTGACTTCAACAGATTCCAC
TGCCACTTTTCACCACGTGACTGGCAAAGACTCATCAACAACAACTGGGGATTCCGACCCAA
GAGACTCAACTTCAAGCTCTTTAACATTCAAGTCAAAGAGGTCACGCAGAATGACGGTACGA
CGACGATTGCCAATAACCTTACCAGCACGGTTCAGGTGTTTACTGACTCGGAGTACCAGCTCC
CGTACGTCCTCGGCTCGGCGCATCAAGGATGCCTCCCGCCGTTCCCAGCAGACGTCTTCATGG
TGCCACAGTATGGATACCTCACCCTGAACAACGGGAGTCAGGCAGTAGGACGCTCTTCATTTT
ACTGCCTGGAGTACTTTCCTTCTCAGATGCTGCGTACCGGAAACAACTTTACCTTCAGCTACA
CTTTTGAGGACGTTCCTTTCCACAGCAGCTACGCTCACAGCCAGAGTCTGGACCGTCTCATGA
ATCCTCTCATCGACCAGTACCTGTATTACTTGAGCAGAACAAACACTCCAAGTGGAACCACCA
CGCAGTCAAGGCTTCAGTTTTCTCAGGCCGGAGCGAGTGACATTCGGGACCAGTCTAGGAAC
TGGCTTCCTGGACCCTGTTACCGCCAGCAGCGAGTATCAAAGACATCTGCGGATAACAACAAC
AGTGAATACTCGTGGACTGGAGCTACCAAGTACCACCTCAATGGCAGAGACTCTCTGGTGAAT
CCGGGCCCGGCGATGGCAAGCCACAAGGACGATGAAGAAAAGTTTTTTCCTCAGAGCGGGG
 
TTCTCATCTTTGGGAAGCAAGGCTCAGAGAAAACAAATGTGGACATTGAAAAGGTCATGATT
ACAGACGAAGAGGAAATCAGGACAACCAATCCCGTGGCTACGGAGCAGTATGGTTCTGTATC
TACCAACCTCCAGAGAGGCAACAGACAAGCAGCTACCGCAGATGTCAACACACAAGGCGTT
CTTCCAGGCATGGTCTGGCAGGACAGAGATGTGTACCTTCAGGGGCCCATCTGGGCAAAGAT
TCCACACACGGACGGACATTTTCACCCCTCTCCCCTCATGGGTGGATTCGGACTTAAACACCC
TCCTCCACAGATTCTCATCAAGAACACCCCGGTACCTGCGAATCCTTCGACCACCTTCAGTGC
GGCAAAGTTTGCTTCCTTCATCACACAGTACTCCACGGGACAGGTCAGCGTGGAGATCGAGT
GGGAGCTGCAGAAGGAAAACAGCAAACGCTGGAATCCCGAAATTCAGTACACTTCCAACTAC
AACAAGTCTGTTAATGTGGACTTTACTGTGGACACTAATGGCGTGTATTCAGAGCCTCGCCCC
ATTGGCACCAGATACCTGACTCGTAATCTGTAA
SEQ ID NO: 5: 
CTGGCGGGGTTTTACGAGATTGTGATTAAGGTCCCCAGCGACCTTGACGAGCATCTGCCCGGC
ATTTCTGACAGCTTTGTGAACTGGGTGGCCGAGAAGGAGTGGGAGTTGCCGCCAGATTCTGA
CTTGGATCTGAATCTGATTGAGCAGGCACCCCTGACCGTGGCCGAGAAGCTGCAGCGCGACT
TTCTGACGGAGTGGCGCCGTGTGAGTAAGGCCCCGGAGGCCCTTTTCTTTGTGCAATTTGAGA
AGGGAGAGAGCTACTTCCACTTACACGTGCTCGTGGAAACCACCGGGGTGAAATCCTTAGTT
TTGGGACGTTTCCTGAGTCAGATTCGCGAAAAACTGATTCAGAGAATTTACCGCGGGATCGAG
CCGACTTTGCCAAACTGGTTCGCGGTCACAAAGACCAGAAACGGCGCCGGAGGCGGGAACA
AGGTGGTGGACGAGTGCTACATCCCCAATTACTTGCTCCCCAAAACCCAGCCTGAGCTCCAGT
GGGCGTGGACTAATTTAGAACAGTATTTAAGCGCCTGTTTGAATCTCACGGAGCGTAAACGGT
TGGTGGCGCAGCATCTGACGCACGTGTCGCAGACGCAGGAGCAGAACAAAGAGAATCAGAA
TCCCAATTCTGACGCGCCGGTGATCAGATCAAAAACTTCAGCCAGGTACATGGAGCTGGTCG
GGTGGCTCGTGGACAAGGGGATTACCTCGGAGAAGCAGTGGATACAGGAGGACCAGGCCTC
ATACATCTCCTTCAATGCGGCCTCCAACTCGCGGTCCCAAATCAAGGCTGCCTTGGACAATGC
GGGAAAGATTATGAGCCTGACTAAAACCGCCCCCGACTACCTGGTGGGCCAGCAGCCCGTGG
AGGACATTTCCAGCAATCGGATTTATAAAATTTTGGAACTAAACGGGTACGATCCCCAATATGC
GGCTTCCGTCTTTCTGGGATGGGCCACGAAAAAGTTCGGCAAGAGGAACACCATCTGGCTGT
TTGGGCCTGCAACTACCGGGAAGACCAACATCGCGGAGGCCATAGCCCACACTGTGCCCTTC
TACGGGTGCGTAAACTGGACCAATGAGAACTTTCCCTTCAACGACTGTGTGGACAAGATGGT
GATCTGGTGGGAGGAGGGGAAGATGACCGCCAAGGTCGTGGAGTCGGCCAAAGCCATTCTC
GGAGGAAGCAAGGTGCGCGTGGACCAGAAATGCAAGTCCTCGGCCCAGATAGACCCGACTC
CCGTGATCGTCACCTCCAACACCAACATGTGCGCCGTGATTGACGGGAACTCAACGACCTTC
GAACACCAGCAGCCGTTGCAAGACCGGATGTTCAAATTTGAACTCACCCGCCGTCTGGATCA
 
TGACTTTGGGAAGGTCACCAAGCAGGAAGTCAAAGACTTTTTCCGGTGGGCAAAGGATCAC
GTGGTTGAGGTGGAGCATGAATTCTACGTCAAAAAGGGTGGAGCCAAGAAAAGACCCGCCC
CCAGTGACGCAGATATAAGTGAGCCCAAACGGGTGCGCGAGTCAGTTGCGCAGCCATCGACG
TCAGACGCGGAAGCATCGATCAACTACGCAGACAGGTACCAAAACAAATGTTCTCGTCACGT
GGGCATGAATCTGATGCTGTTTCCCTGCAGACAATGCGAGAGAATGAATCAGAATTCAAATAT
CTGCTTCACTCACGGACAGAAAGACTGTTTAGAGTGCTTTCCCGTGTCAGAATCTCAACCCGT
TTCTGTCGTCAAAAAGGCGTATCAGAAACTGTGCTACATTCATCATATCATGGGAAAGGTGCC
AGACGCTTGCACTGCCTGCGATCTGGTCAATGTGGATTTGGATGACTGCATCTTTGAACAATA
A
SEQ ID NO: 6: 
ATCGATGTTGACCCCAACAAAAGATTTATAATTAATCATAATCACGAACAACAACAAGTCAATG
AAACAAATAAACAAGTTGTCGATAAAACATTCATAAATGACACAGCAACATACAATTCTTGCA
TAATAAAAATTTAAATGACATCATATTTGAGAATAACAAATGACATTATCCCTCGATTGTGTTTT
ACAAGTAGAATTCTACCCGTAAAGCGAGTTTAGTTTTGAAAAACAAATGACATCATTTGTATAA
TGACATCATCCCCTGATTGTGTTTTACAAGTAGAATTCTATCCGTAAAGCGAGTTCAGTTTTGA
AAACAAATGAGTCATACCTAAACACGTTAATAATCTTCTGATATCAGCTTATGACTCAAGTTAT
GAGCCGTGTGCAAAACATGAGATAAGTTTATGACATCATCCACTGATCGTGCGTTACAAGTAG
AATTCTACTCGTAAAGCCAGTTCGGTTATGAGCCGTGTGCAAAACATGACATCAGCTTATGAC
TCATACTTGATTGTGTTTTACGCGTAGAATTCTACTCGTAAAGCGAGTTCGGTTATGAGCCGTG
TGCAAAACATGACATCAGCTTATGAGTCATAATTAATCGTGCGTTACAAGTAGAATTCTACTCG
TAAAGCGAGTTGAAGGATCATATTTAGTTGCGTTTATGAGATAAGATTGAAAGCACGTGTAAA
ATGTTTCCCGCGCGTTGGCACAACTATTTACAATGCGGCCAAGTTATAAAAGATTCTAATCTGA
TATGTTTTAAAACACCTTTGCGGCCCGAGTTGTTTGCGTACGTGACTAGCGAAGAAGATGTGT
GGACCGCAGAACAGATAGTAAAACAAAACCCTAGTATTGGAGCAATAATCGATGACGTCGCT
GACGTAGCGCTTTGAATTCCGCGCGCTTCGGACCGGGATCGTGACCAAATTCCGTTTTGCGAC
GATGCAGAGTTTTTGAACAGGCTGCTCAAACACATAGATCCGTACCCGCTCAGTCGGATGTAT
TACAATGCAGCCAATACCATGTTTTACACGACTATGGAAAACTATGCCGTGTCCAATTGCAAGT
TCAACATTGAGGATTACAATAACATATTTAAGGTGATGGAAAATATTAGGAAACACAGCAACA
AAAATTCAAACGACCAAGACGAGTTAAACATATATTTGGGAGTTCAGTCGTCGAATGCAAAGC
GTAAAAAATATTAATAAGGTAAAAATTACAGCTACATAAATTACACAATTTAAACTGCAGTCTG
GAGATACGGACCTTTAATTCAACCCAACACAATATATTATAGTTAAATAAGAATTATTATCAAAT
CATTTGTATATTAATTAAAATACTATACTGTAAATTACATTTTATTTACAATC
SEQ ID NO: 7: 
AACTTGTTTATTGCAGCTTATAATGGTTACAAATAAAGCAATAGCATCACAAATTTCACAAATA
AAGCATTTTTTTCACTGCATTCTAGTTGTGGTTTGTCCAAACTCATCAATGTATCTTATCATGTC
TGGATCTGATCACTGCTTGAGCCTAGGAGATCCGATCATGGAGATAATTAAAATGATAACCATC
TCGCAAATAAATAAGTATTTTACTGTTTTCGTAACAGTTTTGTAATAAAAAAACCTATAAATATT
CCGGATTATTCATACCGTCCCACCATCGGGCGCATGATCCGTACATCCAGCCACGTCCTGAACG
TCCAAGAAAACATCATGACTTCCAACTGTGCTTCCAGCCCCTACTCCTGTGAGGCCACTTCAG
CCTGCGCTGAGGCCCAGCAACTGCAGGTGGACACAGGTGGCGATAAGATCGTGAACAACCA
GGTCACCATGACTCAAATCAACTTCAACGCTTCCTACACCTCTGCCAGCACTCCCTCTCGTGC
TAGCTTCGACAACTCATACTCGGAGTTCTGCGACAAGCAACCTAACGATTACTTGTCTTACTAC
AACCACCCAACCCCGGACGGAGCTGATACTGTCATCTCCGACTCTGAAACCGCTGCCGCTAG
CAACTTCCTCGCCTCAGTTAACTCGCTCACTGACAACGATTTGGTGGAGTGTCTGCTCAAGAC
CACTGACAACCTGGAGGAAGCTGTGTCCTCTGCCTACTACAGCGAGTCACTCGAACAGCCAG
TGGTCGAACAACCCTCTCCTAGCTCAGCTTACCACGCCGAGTCCTTCGAACACTCTGCTGGTG
TCAACCAGCCGTCGGCCACAGGCACCAAGAGGAAGTTGGACGAGTACCTGGATAACTCCCA
GGGAGTTGTGGGTCAATTCAACAAGATCAAGTTGAGACCTAAGTACAAGAAGAGCACCATCC
AGTCATGCGCTACACTGGAACAAACCATCAACCACAACACTAACATCTGTACAGTGGCTTCCA
CCCAGGAGATCACTCACTACTTCACAAACGACTTCGCCCCCTACCTGATGAGGTTCGACGATA
ACGACTACAACTCGAACAGATTCTCCGATCACATGTCTGAAACCGGTTACTACATGTTCGTCG
TTAAGAAGTCCGAGGTGAAGCCTTTCGAAATCATCTTCGCCAAGTACGTCTCTAACGTGGTCT
ACGAGTACACAAACAACTACTACATGGTTGACAACCGTGTGTTCGTTGTGACCTTCGATAAGA
TCCGCTTCATGATCAGCTACAACCTGGTTAAGGAGACTGGCATCGAAATCCCACACTCACAGG
ACGTCTGCAACGATGAGACCGCCGCTCAAAACTGCAAGAAGTGTCACTTCGTGGACGTCCAC
CACACATTCAAGGCCGCTCTGACCTCCTACTTCAACCTCGATATGTACTACGCTCAGACAACC
TTCGTGACCTTGCTGCAATCACTCGGCGAGCGTAAGTGTGGATTCCTCTTGTCGAAGTTGTAC
GAGATGTACCAGGACAAGAACCTCTTCACTTTGCCCATCATGCTGAGCCGCAAGGAATCAAA
CGAGATCGAAACCGCCTCTAACAACTTCTTCGTCTCGCCATACGTTTCCCAGATCCTCAAGTA
CTCGGAGTCCGTCCAATTCCCGGACAACCCTCCCAACAAGTACGTCGTTGATAACCTGAACCT
CATCGTGAACAAGAAGAGCACTCTGACATACAAGTACTCGTCCGTCGCTAACCTGCTCTTCAA
CAACTACAAGTACCACGACAACATCGCTTCTAACAACAACGCCGAGAACCTCAAGAAGGTCA
AGAAGGAAGACGGAAGCATGCACATCGTTGAGCAGTACTTGACTCAAAACGTCGATAACGTT
AAGGGTCACAACTTCATCGTGTTGTCCTTCAAGAACGAGGAAAGGCTGACCATCGCTAAGAA
GAACAAGGAGTTCTACTGGATCTCTGGCGAAATCAAGGACGTTGATGTGAGCCAGGTCATCC
AAAAGTACAACAGATTCAAGCACCACATGTTCGTGATCGGCAAGGTCAACCGTCGCGAGTCA
 
ACTACACTGCACAACAACTTGCTGAAGCTCTTGGCCTTGATCCTGCAGGGACTGGTGCCACTC
TCCGACGCCATCACATTCGCCGAGCAAAAGCTCAACTGCAAGTACAAGAAGTTCGAGTTCAA
CTAA
SEQ ID NO: 9: 
TTCTCTGTCACAGAATGAAAATTTTTCTGTCATCTCTTCGTTATTAATGTTTGTAATTGACTGAA
TATCAACGCTTATTTGCAGCCTGAATGGCGAATGGGACGCGCCCTGTAGCGGCGCATTAAGCG
CGGCGGGTGTGGTGGTTACGCGCAGCGTGACCGCTACACTTGCCAGCGCCCTAGCGCCCGCT
CCTTTCGCTTTCTTCCCTTCCTTTCTCGCCACGTTCGCCGGCTTTCCCCGTCAAGCTCTAAATC
GGGGGCTCCCTTTAGGGTTCCGATTTAGTGCTTTACGGCACCTCGACCCCAAAAAACTTGATT
AGGGTGATGGTTCACGTAGTGGGCCATCGCCCTGATAGACGGTTTTTCGCCCTTTGACGTTGG
AGTCCACGTTCTTTAATAGTGGACTCTTGTTCCAAACTGGAACAACACTCAACCCTATCTCGG
TCTATTCTTTTGATTTATAAGGGATTTTGCCGATTTCGGCCTATTGGTTAAAAAATGAGCTGATT
TAACAAAAATTTAACGCGAATTTTAACAAAATATTAACGTTTACAATTTCAGGTGGCACTTTTC
GGGGAAATGTGCGCGGAACCCCTATTTGTTTATTTTTCTAAATACATTCAAATATGTATCCGCTC
ATGAGACAATAACCCTGATAAATGCTTCAATAATATTGAAAAAGGAAGAGTATGAGTATTCAAC
ATTTCCGTGTCGCCCTTATTCCCTTTTTTGCGGCATTTTGCCTTCCTGTTTTTGCTCACCCAGAA
ACGCTGGTGAAAGTAAAAGATGCTGAAGATCAGTTGGGTGCACGAGTGGGTTACATCGAACT
GGATCTCAACAGCGGTAAGATCCTTGAGAGTTTTCGCCCCGAAGAACGTTTTCCAATGATGAG
CACTTTTAAAGTTCTGCTATGTGGCGCGGTATTATCCCGTATTGACGCCGGGCAAGAGCAACTC
GGTCGCCGCATACACTATTCTCAGAATGACTTGGTTGAGTACTCACCAGTCACAGAAAAGCAT
CTTACGGATGGCATGACAGTAAGAGAATTATGCAGTGCTGCCATAACCATGAGTGATAACACT
GCGGCCAACTTACTTCTGACAACGATCGGAGGACCGAAGGAGCTAACCGCTTTTTTGCACAA
CATGGGGGATCATGTAACTCGCCTTGATCGTTGGGAACCGGAGCTGAATGAAGCCATACCAAA
CGACGAGCGTGACACCACGATGCCTGTAGCAATGGCAACAACGTTGCGCAAACTATTAACTG
GCGAACTACTTACTCTAGCTTCCCGGCAACAATTAATAGACTGGATGGAGGCGGATAAAGTTG
CAGGACCACTTCTGCGCTCGGCCCTTCCGGCTGGCTGGTTTATTGCTGATAAATCTGGAGCCG
GTGAGCGTGGGTCTCGCGGTATCATTGCAGCACTGGGGCCAGATGGTAAGCCCTCCCGTATCG
TAGTTATCTACACGACGGGGAGTCAGGCAACTATGGATGAACGAAATAGACAGATCGCTGAGA
TAGGTGCCTCACTGATTAAGCATTGGTAACTGTCAGACCAAGTTTACTCATATATACTTTAGATT
GATTTAAAACTTCATTTTTAATTTAAAAGGATCTAGGTGAAGATCCTTTTTGATAATCTCATGAC
CAAAATCCCTTAACGTGAGTTTTCGTTCCACTGAGCGTCAGACCCCGTAGAAAAGATCAAAG
GATCTTCTTGAGATCCTTTTTTTCTGCGCGTAATCTGCTGCTTGCAAACAAAAAAACCACCGCT
ACCAGCGGTGGTTTGTTTGCCGGATCAAGAGCTACCAACTCTTTTTCCGAAGGTAACTGGCTT
 
CAGCAGAGCGCAGATACCAAATACTGTCCTTCTAGTGTAGCCGTAGTTAGGCCACCACTTCAA
GAACTCTGTAGCACCGCCTACATACCTCGCTCTGCTAATCCTGTTACCAGTGGCTGCTGCCAGT
GGCGATAAGTCGTGTCTTACCGGGTTGGACTCAAGACGATAGTTACCGGATAAGGCGCAGCG
GTCGGGCTGAACGGGGGGTTCGTGCACACAGCCCAGCTTGGAGCGAACGACCTACACCGAA
CTGAGATACCTACAGCGTGAGCATTGAGAAAGCGCCACGCTTCCCGAAGGGAGAAAGGCGG
ACAGGTATCCGGTAAGCGGCAGGGTCGGAACAGGAGAGCGCACGAGGGAGCTTCCAGGGGG
AAACGCCTGGTATCTTTATAGTCCTGTCGGGTTTCGCCACCTCTGACTTGAGCGTCGATTTTTG
TGATGCTCGTCAGGGGGGCGGAGCCTATGGAAAAACGCCAGCAACGCGGCCTTTTTACGGTT
CCTGGCCTTTTGCTGGCCTTTTGCTCACATGTTCTTTCCTGCGTTATCCCCTGATTCTGTGGATA
ACCGTATTACCGCCTTTGAGTGAGCTGATACCGCTCGCCGCAGCCGAACGACCGAGCGCAGC
GAGTCAGTGAGCGAGGAAGCGGAAGAGCGCCTGATGCGGTATTTTCTCCTTACGCATCTGTG
CGGTATTTCACACCGCAGACCAGCCGCGTAACCTGGCAAAATCGGTTACGGTTGAGTAATAAA
TGGATGCCCTGCGTAAGCGGGTGTGGGCGGACAATAAAGTCTTAAACTGAACAAAATAGATCT
AAACTATGACAATAAAGTCTTAAACTAGACAGAATAGTTGTAAACTGAAATCAGTCCAGTTAT
GCTGTGAAAAAGCATACTGGACTTTTGTTATGGCTAAAGCAAACTCTTCATTTTCTGAAGTGC
AAATTGCCCGTCGTATTAAAGAGGGGCGTGGCCAAGGGCATGGTAAAGACTATATTCGCGGCG
TTGTGACAATTTACCGAACAACTCCGCGGCCGGGAAGCCGATCTCGGCTTGAACGAATTGTTA
GGTGGCGGTACTTGGGTCGATATCAAAGTGCATCACTTCTTCCCGTATGCCCAACTTTGTATAG
AGAGCCACTGCGGGATCGTCACCGTAATCTGCTTGCACGTAGATCACATAAGCACCAAGCGCG
TTGGCCTCATGCTTGAGGAGATTGATGAGCGCGGTGGCAATGCCCTGCCTCCGGTGCTCGCCG
GAGACTGCGAGATCATAGATATAGATCTCACTACGCGGCTGCTCAAACCTGGGCAGAACGTAA
GCCGCGAGAGCGCCAACAACCGCTTCTTGGTCGAAGGCAGCAAGCGCGATGAATGTCTTACT
ACGGAGCAAGTTCCCGAGGTAATCGGAGTCCGGCTGATGTTGGGAGTAGGTGGCTACGTCTC
CGAACTCACGACCGAAAAGATCAAGAGCAGCCCGCATGGATTTGACTTGGTCAGGGCCGAGC
CTACATGTGCGAATGATGCCCATACTTGAGCCACCTAACTTTGTTTTAGGGCGACTGCCCTGCT
GCGTAACATCGTTGCTGCTGCGTAACATCGTTGCTGCTCCATAACATCAAACATCGACCCACG
GCGTAACGCGCTTGCTGCTTGGATGCCCGAGGCATAGACTGTACAAAAAAACAGTCATAACA
AGCCATGAAAACCGCCACTGCGCCGTTACCACCGCTGCGTTCGGTCAAGGTTCTGGACCAGT
TGCGTGAGCGCATACGCTACTTGCATTACAGTTTACGAACCGAACAGGCTTATGTCAACTGGG
TTCGTGCCTTCATCCGTTTCCACGGTGTGCGTCACCCGGCAACCTTGGGCAGCAGCGAAGTC
GAGGCATTTCTGTCCTGGCTGGCGAACGAGCGCAAGGTTTCGGTCTCCACGCATCGTCAGGC
ATTGGCGGCCTTGCTGTTCTTCTACGGCAAGGTGCTGTGCACGGATCTGCCCTGGCTTCAGGA
GATCGGTAGACCTCGGCCGTCGCGGCGCTTGCCGGTGGTGCTGACCCCGGATGAAGTGGTTC
GCATCCTCGGTTTTCTGGAAGGCGAGCATCGTTTGTTCGCCCAGGACTCTAGCTATAGTTCTAG
 
TGGTTGGCCTACGTACCCGTAGTGGCTATGGCAGGGCTTGCCGCCCCGACGTTGGCTGCGAGC
CCTGGGCCTTCACCCGAACTTGGGGGTTGGGGTGGGGAAAAGGAAGAAACGCGGGCGTATT
GGTCCCAATGGGGTCTCGGTGGGGTATCGACAGAGTGCCAGCCCTGGGACCGAACCCCGCGT
TTATGAACAAACGACCCAACACCCGTGCGTTTTATTCTGTCTTTTTATTGCCGTCATAGCGCGG
GTTCCTTCCGGTATTGTCTCCTTCCGTGTTTCAGTTAGCCTCCCCCATCTCCCGGTACCGCATGC
TTACAGATTACGAGTCAGGTATCTGGTGCCAATGGGGCGAGGCTCTGAATACACGCCATTAGT
GTCCACAGTAAAGTCCACATTAACAGACTTGTTGTAGTTGGAAGTGTACTGAATTTCGGGATT
CCAGCGTTTGCTGTTTTCCTTCTGCAGCTCCCACTCGATCTCCACGCTGACCTGTCCCGTGGA
GTACTGTGTGATGAAGGAAGCAAACTTTGCCGCACTGAAGGTGGTCGAAGGATTCGCAGGTA
CCGGGGTGTTCTTGATGAGAATCTGTGGAGGAGGGTGTTTAAGTCCGAATCCACCCATGAGG
GGAGAGGGGTGAAAATGTCCGTCCGTGTGTGGAATCTTTGCCCAGATGGGCCCCTGAAGGTA
CACATCTCTGTCCTGCCAGACCATGCCTGGAAGAACGCCTTGTGTGTTGACATCTGCGGTAGC
TGCTTGTCTGTTGCCTCTCTGGAGGTTGGTAGATACAGAACCATACTGCTCCGTAGCCACGGG
ATTGGTTGTCCTGATTTCCTCTTCGTCTGTAATCATGACCTTTTCAATGTCCACATTTGTTTTCT
CTGAGCCTTGCTTCCCAAAGATGAGAACCCCGCTCTGAGGAAAAAACTTTTCTTCATCGTCCT
TGTGGCTTGCCATCGCCGGGCCCGGATTCACCAGAGAGTCTCTGCCATTGAGGTGGTACTTGG
TAGCTCCAGTCCACGAGTATTCACTGTTGTTGTTATCCGCAGATGTCTTTGATACTCGCTGCTG
GCGGTAACAGGGTCCAGGAAGCCAGTTCCTAGACTGGTCCCGAATGTCACTCGCTCCGGCCT
GAGAAAACTGAAGCCTTGACTGCGTGGTGGTTCCACTTGGAGTGTTTGTTCTGCTCAAGTAAT
ACAGGTACTGGTCGATGAGAGGATTCATGAGACGGTCCAGACTCTGGCTGTGAGCGTAGCTG
CTGTGGAAAGGAACGTCCTCAAAAGTGTAGCTGAAGGTAAAGTTGTTTCCGGTACGCAGCAT
CTGAGAAGGAAAGTACTCCAGGCAGTAAAATGAAGAGCGTCCTACTGCCTGACTCCCGTTGT
TCAGGGTGAGGTATCCATACTGTGGCACCATGAAGACGTCTGCTGGGAACGGCGGGAGGCAT
CCTTGATGCGCCGAGCCGAGGACGTACGGGAGCTGGTACTCCGAGTCAGTAAACACCTGAAC
CGTGCTGGTAAGGTTATTGGCAATCGTCGTCGTACCGTCATTCTGCGTGACCTCTTTGACTTGA
ATGTTAAAGAGCTTGAAGTTGAGTCTCTTGGGTCGGAATCCCCAGTTGTTGTTGATGAGTCTT
TGCCAGTCACGTGGTGAAAAGTGGCAGTGGAATCTGTTGAAGTCAAAATACCCCCAAGGGGT
GCTGTAGCCAAAGTAGTGATTGTCGTTCGAGGCTCCTGATTGGCTGGAAATTTGTTTGTAGAG
GTGGTTGTTGTAGGTGGGCAGGGCCCAGGTTCGGGTGCTGGTGGTGATGACTCTGTCGCCCAT
CCATGTGGAATCGCAATGCCAATTTCCCGAGGAATTACCCACTCCGTCGGCGCCCTCGTTATTG
TCTGCCATTGGTGCGCCACTGCCTGTAGCCATCGTATTAGTTCCCAGACCAGAGGGGGCTGCT
GGTGGCTGTCCGAGAGGCTGGGGGTCAGGTACTGAGTCTGCGTCTCCAGTCTGACCAAAATT
CAATCTTTTTCTTGCAGGCTGCTGGCCCGCCTTTCCGGTTCCCGAGGAGGAGTCTGGCTCCAC
AGGAGAGTGCTCTACCGGCCTCTTTTTTCCCGGAGCCGTCTTAACAGGTTCCTCAACCAGGCC
 
CAGAGGTTCAAGAACCCTCTTTTTCGCCTGGAAGACTGCTCGTCCGAGGTTGCCCCCAAAAG
ACGTATCTTCTTTAAGGCGCTCCTGGAACTCCGCGTCGGCGTGGTTGTACTTGAGGTAAGGGT
TGTCTCCGCTGTCGAGCTGCCGGTCGTAGGCTTTGTCGTGCTCGAGGGCAGCGGCGTCTGCCT
CGTTCACCGGCTCTCCCTTGTCCAGTCCGTTGAAGGGTCCGAGGTACTTGTACCCGGGAAGC
ACAAGGCCCCTGCTGTCGTCCTTATGCCGCTCTGCGGGCTTTGGTGGTGGTGGGCCGGGTTTG
AGCTTCCACCACTGTCTTATTCCTTCAGAGAGAGTGTCCTCGAGCCAATCGGGTAGATAACCG
TCGGCAGCCGTGGCGGCGCTAGCACCATGGCTCGAGATCCCGGGTGATCAAGTCTTCGTCGA
GTGATTGTAAATAAAATGTAATTTACAGTATAGTATTTTAATTAATATACAAATGATTTGATAATA
ATTCTTATTTAACTATAATATATTGTGTTGGGTTGAATTAAAGGTCCGTATACGCCGCTACAGGG
CGCGTACTATGGTTGCTTTGACGTATGCGGTGTGAAATACCGCACAGATGCGTAAGGAGAAAA
TACCGCATCAGGCGCCCCTGCAGGCAGCTGCGCGCTCGCTCGCTCACTGAGGCCGCCCGGGC
AAAGCCCGGGCGTCGGGCGACCTTTGGTCGCCCGGCCTCAGTGAGCGAGCGAGCGCGCAGA
GAGGGAGTGGCCAACTCCATCACTAGGGGTTCCTACGCGTTAGTTATTAATAGTAATCAATTAC
GGGGTCATTAGTTCATAGCCCATATATGGAGTTCCGCGTTACATAACTTACGGTAAATGGCCCG
CCTGGCTGACCGCCCAACGACCCCCGCCCATTGACGTCAATAATGACGTATGTTCCCATAGTA
ACGCCAATAGGGACTTTCCATTGACGTCAATGGGTGGAGTATTTACGGTAAACTGCCCACTTG
GCAGTACATCAAGTGTATCATATGCCAAGTACGCCCCCTATTGACGTCAATGACGGTAAATGGC
CCGCCTGGCATTATGCCCAGTACATGACCTTATGGGACTTTCCTACTTGGCAGTACATCTACGT
ATTAGTCATCGCTATTACCATGGTGATGCGGTTTTGGCAGTACATCAATGGGCGTGGATAGCGG
TTTGACTCACGGGGATTTCCAAGTCTCCACCCCATTGACGTCAATGGGAGTTTGTTTTGGCAC
CAAAATCAACGGGACTTTCCAAAATGTCGTAACAACTCCGCCCCATTGACGCAAATGGGCGG
TAGGCGTGTACGGTGGGAGGTCTATATAAGCAGAGCTGGTTTAGTGAACCGTCAGATCCGCTA
GCGCTACCGGTCGCCACCATGGTGAGCAAGGGCGAGGAGCTGTTCACCGGGGTGGTGCCCAT
CCTGGTCGAGCTGGACGGCGACGTAAACGGCCACAAGTTCAGCGTGTCCGGCGAGGGCGAG
GGCGATGCCACCTACGGCAAGCTGACCCTGAAGTTCATCTGCACCACCGGCAAGCTGCCCGT
GCCCTGGCCCACCCTCGTGACCACCCTGACCTACGGCGTGCAGTGCTTCAGCCGCTACCCCG
ACCACATGAAGCAGCACGACTTCTTCAAGTCCGCCATGCCCGAAGGCTACGTCCAGGAGCGC
ACCATCTTCTTCAAGGACGACGGCAACTACAAGACCCGCGCCGAGGTGAAGTTCGAGGGCG
ACACCCTGGTGAACCGCATCGAGCTGAAGGGCATCGACTTCAAGGAGGACGGCAACATCCTG
GGGCACAAGCTGGAGTACAACTACAACAGCCACAACGTCTATATCATGGCCGACAAGCAGAA
GAACGGCATCAAGGTGAACTTCAAGATCCGCCACAACATCGAGGACGGCAGCGTGCAGCTC
GCCGACCACTACCAGCAGAACACCCCCATCGGCGACGGCCCCGTGCTGCTGCCCGACAACCA
CTACCTGAGCACCCAGTCCGCCCTGAGCAAAGACCCCAACGAGAAGCGCGATCACATGGTCC
TGCTGGAGTTCGTGACCGCCGCCGGGATCACTCTCGGCATGGACGAGCTGTACAAGTAGGTC
 
GACGTTTATTTGTGAAATTTGTGATGCTATTGCTTTATTTGTAACCATCTAGCTTTATTTGTGAAA
TTTGTGATGCTATTGCTTTATTTGTAACCATTATAAGCTGCAATAAACAAGTTAACAACAACAAT
TGCATTCATTTTATGTTTCAGGTTCAGGGGGAGATGTGGGAGGTTTTTTAAAGTTTAAACAGG
AACCCCTAGTGATGGAGTTGGCCACTCCCTCTCTGCGCGCTCGCTCGCTCACTGAGGCCGGGC
GACCAAAGGTCGCCCGACGCCCGGGCGGCCTCAGTGAGCGAGCGAGCGCGCAGCTGCCTGC
AGGACATGTGAGCAAAAGGCCAGCAAAAGGCCAGGAACCGTAAAAAGGCCGCGTTGCTGGC
GTTTTTCCATAGGCTCCGCCCCCCTGACGAGCATCACAAAAATCGACGCTCGCGGCCGCTCTA
GAGATCATGGAGATAATTAAAATGATAACCATCTCGCAAATAAATAAGTATTTTACTGTTTTCGT
AACAGTTTTGTAATAAAAAAACCTATAAATATTCCGGATTATTCATACCGTCCCACCATCGGGC
GCGGATCCGCCGCCCTGGCGGGGTTTTACGAGATTGTGATTAAGGTCCCCAGCGACCTTGACG
AGCATCTGCCCGGCATTTCTGACAGCTTTGTGAACTGGGTGGCCGAGAAGGAGTGGGAGTTG
CCGCCAGATTCTGACTTGGATCTGAATCTGATTGAGCAGGCACCCCTGACCGTGGCCGAGAA
GCTGCAGCGCGACTTTCTGACGGAGTGGCGCCGTGTGAGTAAGGCCCCGGAGGCCCTTTTCT
TTGTGCAATTTGAGAAGGGAGAGAGCTACTTCCACTTACACGTGCTCGTGGAAACCACCGGG
GTGAAATCCTTAGTTTTGGGACGTTTCCTGAGTCAGATTCGCGAAAAACTGATTCAGAGAATT
TACCGCGGGATCGAGCCGACTTTGCCAAACTGGTTCGCGGTCACAAAGACCAGAAACGGCGC
CGGAGGCGGGAACAAGGTGGTGGACGAGTGCTACATCCCCAATTACTTGCTCCCCAAAACCC
AGCCTGAGCTCCAGTGGGCGTGGACTAATTTAGAACAGTATTTAAGCGCCTGTTTGAATCTCA
CGGAGCGTAAACGGTTGGTGGCGCAGCATCTGACGCACGTGTCGCAGACGCAGGAGCAGAA
CAAAGAGAATCAGAATCCCAATTCTGACGCGCCGGTGATCAGATCAAAAACTTCAGCCAGGT
ACATGGAGCTGGTCGGGTGGCTCGTGGACAAGGGGATTACCTCGGAGAAGCAGTGGATACAG
GAGGACCAGGCCTCATACATCTCCTTCAATGCGGCCTCCAACTCGCGGTCCCAAATCAAGGCT
GCCTTGGACAATGCGGGAAAGATTATGAGCCTGACTAAAACCGCCCCCGACTACCTGGTGGG
CCAGCAGCCCGTGGAGGACATTTCCAGCAATCGGATTTATAAAATTTTGGAACTAAACGGGTA
CGATCCCCAATATGCGGCTTCCGTCTTTCTGGGATGGGCCACGAAAAAGTTCGGCAAGAGGA
ACACCATCTGGCTGTTTGGGCCTGCAACTACCGGGAAGACCAACATCGCGGAGGCCATAGCC
CACACTGTGCCCTTCTACGGGTGCGTAAACTGGACCAATGAGAACTTTCCCTTCAACGACTGT
GTGGACAAGATGGTGATCTGGTGGGAGGAGGGGAAGATGACCGCCAAGGTCGTGGAGTCGG
CCAAAGCCATTCTCGGAGGAAGCAAGGTGCGCGTGGACCAGAAATGCAAGTCCTCGGCCCA
GATAGACCCGACTCCCGTGATCGTCACCTCCAACACCAACATGTGCGCCGTGATTGACGGGA
ACTCAACGACCTTCGAACACCAGCAGCCGTTGCAAGACCGGATGTTCAAATTTGAACTCACC
CGCCGTCTGGATCATGACTTTGGGAAGGTCACCAAGCAGGAAGTCAAAGACTTTTTCCGGTG
GGCAAAGGATCACGTGGTTGAGGTGGAGCATGAATTCTACGTCAAAAAGGGTGGAGCCAAG
AAAAGACCCGCCCCCAGTGACGCAGATATAAGTGAGCCCAAACGGGTGCGCGAGTCAGTTGC
 
GCAGCCATCGACGTCAGACGCGGAAGCATCGATCAACTACGCAGACAGGTACCAAAACAAAT
GTTCTCGTCACGTGGGCATGAATCTGATGCTGTTTCCCTGCAGACAATGCGAGAGAATGAATC
AGAATTCAAATATCTGCTTCACTCACGGACAGAAAGACTGTTTAGAGTGCTTTCCCGTGTCAG
AATCTCAACCCGTTTCTGTCGTCAAAAAGGCGTATCAGAAACTGTGCTACATTCATCATATCAT
GGGAAAGGTGCCAGACGCTTGCACTGCCTGCGATCTGGTCAATGTGGATTTGGATGACTGCAT
CTTTGAACAATAATCTAGAGCCTGCAGTCTCGACAAGCTTGTCGAGAAGTACTAGAGGATCAT
AATCAGCCATACCACATTTGTAGAGGTTTTACTTGCTTTAAAAAACCTCCCACACCTCCCCCTG
AACCTGAAACATAAAATGAATGCAATTGTTGTTGTTAACTTGTTTATTGCAGCTTATAATGGTTA
CAAATAAAGCAATAGCATCACAAATTTCACAAATAAAGCATTTTTTTCACTGCATTCTAGTTGT
GGTTTGTCCAAACTCATCAATGTATCTTATCATGTCTGGATCTGATCACTGCTTGAGCCTAGGA
GATCCGAACCAGATAAGTGAAATCTAGTTCCAAACTATTTTGTCATTTTTAATTTTCGTATTAGC
TTACGACGCTACACCCAGTTCCCATCTATTTTGTCACTCTTCCCTAAATAATCCTTAAAAACTCC
ATTTCCACCCCTCCCAGTTCCCAACTATTTTGTCCGCCCACAGCGGGGCATTTTTCTTCCTGTT
ATGTTTTTAATCAAACATCCTGCCAACTCCATGTGACAAACCGTCATCTTCGGCTACTTT
SEQ ID NO: 9:

TTCTCTGTCACAGAATGAAAATTTTTCTGTCATCTCTTCGTTATTAATGTTTGTAATTGACTGAA
TATCAACGCTTATTTGCAGCCTGAATGGCGAATGGGACGCGCCCTGTAGCGGCGCATTAAGCG
CGGCGGGTGTGGTGGTTACGCGCAGCGTGACCGCTACACTTGCCAGCGCCCTAGCGCCCGCT
CCTTTCGCTTTCTTCCCTTCCTTTCTCGCCACGTTCGCCGGCTTTCCCCGTCAAGCTCTAAATC
GGGGGCTCCCTTTAGGGTTCCGATTTAGTGCTTTACGGCACCTCGACCCCAAAAAACTTGATT
AGGGTGATGGTTCACGTAGTGGGCCATCGCCCTGATAGACGGTTTTTCGCCCTTTGACGTTGG
AGTCCACGTTCTTTAATAGTGGACTCTTGTTCCAAACTGGAACAACACTCAACCCTATCTCGG
TCTATTCTTTTGATTTATAAGGGATTTTGCCGATTTCGGCCTATTGGTTAAAAAATGAGCTGATT
TAACAAAAATTTAACGCGAATTTTAACAAAATATTAACGTTTACAATTTCAGGTGGCACTTTTC
GGGGAAATGTGCGCGGAACCCCTATTTGTTTATTTTTCTAAATACATTCAAATATGTATCCGCTC
ATGAGACAATAACCCTGATAAATGCTTCAATAATATTGAAAAAGGAAGAGTATGAGTATTCAAC
ATTTCCGTGTCGCCCTTATTCCCTTTTTTGCGGCATTTTGCCTTCCTGTTTTTGCTCACCCAGAA
ACGCTGGTGAAAGTAAAAGATGCTGAAGATCAGTTGGGTGCACGAGTGGGTTACATCGAACT
GGATCTCAACAGCGGTAAGATCCTTGAGAGTTTTCGCCCCGAAGAACGTTTTCCAATGATGAG
CACTTTTAAAGTTCTGCTATGTGGCGCGGTATTATCCCGTATTGACGCCGGGCAAGAGCAACTC
GGTCGCCGCATACACTATTCTCAGAATGACTTGGTTGAGTACTCACCAGTCACAGAAAAGCAT
CTTACGGATGGCATGACAGTAAGAGAATTATGCAGTGCTGCCATAACCATGAGTGATAACACT
GCGGCCAACTTACTTCTGACAACGATCGGAGGACCGAAGGAGCTAACCGCTTTTTTGCACAA

CATGGGGGATCATGTAACTCGCCTTGATCGTTGGGAACCGGAGCTGAATGAAGCCATACCAAA
CGACGAGCGTGACACCACGATGCCTGTAGCAATGGCAACAACGTTGCGCAAACTATTAACTG
GCGAACTACTTACTCTAGCTTCCCGGCAACAATTAATAGACTGGATGGAGGCGGATAAAGTTG
CAGGACCACTTCTGCGCTCGGCCCTTCCGGCTGGCTGGTTTATTGCTGATAAATCTGGAGCCG
GTGAGCGTGGGTCTCGCGGTATCATTGCAGCACTGGGGCCAGATGGTAAGCCCTCCCGTATCG
TAGTTATCTACACGACGGGGAGTCAGGCAACTATGGATGAACGAAATAGACAGATCGCTGAGA
TAGGTGCCTCACTGATTAAGCATTGGTAACTGTCAGACCAAGTTTACTCATATATACTTTAGATT
GATTTAAAACTTCATTTTTAATTTAAAAGGATCTAGGTGAAGATCCTTTTTGATAATCTCATGAC
CAAAATCCCTTAACGTGAGTTTTCGTTCCACTGAGCGTCAGACCCCGTAGAAAAGATCAAAG
GATCTTCTTGAGATCCTTTTTTTCTGCGCGTAATCTGCTGCTTGCAAACAAAAAAACCACCGCT
ACCAGCGGTGGTTTGTTTGCCGGATCAAGAGCTACCAACTCTTTTTCCGAAGGTAACTGGCTT
CAGCAGAGCGCAGATACCAAATACTGTCCTTCTAGTGTAGCCGTAGTTAGGCCACCACTTCAA
GAACTCTGTAGCACCGCCTACATACCTCGCTCTGCTAATCCTGTTACCAGTGGCTGCTGCCAGT
GGCGATAAGTCGTGTCTTACCGGGTTGGACTCAAGACGATAGTTACCGGATAAGGCGCAGCG
GTCGGGCTGAACGGGGGGTTCGTGCACACAGCCCAGCTTGGAGCGAACGACCTACACCGAA
CTGAGATACCTACAGCGTGAGCATTGAGAAAGCGCCACGCTTCCCGAAGGGAGAAAGGCGG
ACAGGTATCCGGTAAGCGGCAGGGTCGGAACAGGAGAGCGCACGAGGGAGCTTCCAGGGGG
AAACGCCTGGTATCTTTATAGTCCTGTCGGGTTTCGCCACCTCTGACTTGAGCGTCGATTTTTG
TGATGCTCGTCAGGGGGGCGGAGCCTATGGAAAAACGCCAGCAACGCGGCCTTTTTACGGTT
CCTGGCCTTTTGCTGGCCTTTTGCTCACATGTTCTTTCCTGCGTTATCCCCTGATTCTGTGGATA
ACCGTATTACCGCCTTTGAGTGAGCTGATACCGCTCGCCGCAGCCGAACGACCGAGCGCAGC
GAGTCAGTGAGCGAGGAAGCGGAAGAGCGCCTGATGCGGTATTTTCTCCTTACGCATCTGTG
CGGTATTTCACACCGCAGACCAGCCGCGTAACCTGGCAAAATCGGTTACGGTTGAGTAATAAA
TGGATGCCCTGCGTAAGCGGGTGTGGGCGGACAATAAAGTCTTAAACTGAACAAAATAGATCT
AAACTATGACAATAAAGTCTTAAACTAGACAGAATAGTTGTAAACTGAAATCAGTCCAGTTAT
GCTGTGAAAAAGCATACTGGACTTTTGTTATGGCTAAAGCAAACTCTTCATTTTCTGAAGTGC
AAATTGCCCGTCGTATTAAAGAGGGGCGTGGCCAAGGGCATGGTAAAGACTATATTCGCGGCG
TTGTGACAATTTACCGAACAACTCCGCGGCCGGGAAGCCGATCTCGGCTTGAACGAATTGTTA
GGTGGCGGTACTTGGGTCGATATCAAAGTGCATCACTTCTTCCCGTATGCCCAACTTTGTATAG
AGAGCCACTGCGGGATCGTCACCGTAATCTGCTTGCACGTAGATCACATAAGCACCAAGCGCG
TTGGCCTCATGCTTGAGGAGATTGATGAGCGCGGTGGCAATGCCCTGCCTCCGGTGCTCGCCG
GAGACTGCGAGATCATAGATATAGATCTCACTACGCGGCTGCTCAAACCTGGGCAGAACGTAA
GCCGCGAGAGCGCCAACAACCGCTTCTTGGTCGAAGGCAGCAAGCGCGATGAATGTCTTACT
ACGGAGCAAGTTCCCGAGGTAATCGGAGTCCGGCTGATGTTGGGAGTAGGTGGCTACGTCTC

CGAACTCACGACCGAAAAGATCAAGAGCAGCCCGCATGGATTTGACTTGGTCAGGGCCGAGC
CTACATGTGCGAATGATGCCCATACTTGAGCCACCTAACTTTGTTTTAGGGCGACTGCCCTGCT
GCGTAACATCGTTGCTGCTGCGTAACATCGTTGCTGCTCCATAACATCAAACATCGACCCACG
GCGTAACGCGCTTGCTGCTTGGATGCCCGAGGCATAGACTGTACAAAAAAACAGTCATAACA
AGCCATGAAAACCGCCACTGCGCCGTTACCACCGCTGCGTTCGGTCAAGGTTCTGGACCAGT
TGCGTGAGCGCATACGCTACTTGCATTACAGTTTACGAACCGAACAGGCTTATGTCAACTGGG
TTCGTGCCTTCATCCGTTTCCACGGTGTGCGTCACCCGGCAACCTTGGGCAGCAGCGAAGTC
GAGGCATTTCTGTCCTGGCTGGCGAACGAGCGCAAGGTTTCGGTCTCCACGCATCGTCAGGC
ATTGGCGGCCTTGCTGTTCTTCTACGGCAAGGTGCTGTGCACGGATCTGCCCTGGCTTCAGGA
GATCGGTAGACCTCGGCCGTCGCGGCGCTTGCCGGTGGTGCTGACCCCGGATGAAGTGGTTC
GCATCCTCGGTTTTCTGGAAGGCGAGCATCGTTTGTTCGCCCAGGACTCTAGCTATAGTTCTAG
TGGTTGGCCTACGTACCCGTAGTGGCTATGGCAGGGCTTGCCGCCCCGACGTTGGCTGCGAGC
CCTGGGCCTTCACCCGAACTTGGGGGTTGGGGTGGGGAAAAGGAAGAAACGCGGGCGTATT
GGTCCCAATGGGGTCTCGGTGGGGTATCGACAGAGTGCCAGCCCTGGGACCGAACCCCGCGT
TTATGAACAAACGACCCAACACCCGTGCGTTTTATTCTGTCTTTTTATTGCCGTCATAGCGCGG
GTTCCTTCCGGTATTGTCTCCTTCCGTGTTTCAGTTAGCCTCCCCCATCTCCCGGTACCGCATGC
CTTAGTTGAACTCGAACTTCTTGTACTTGCAGTTGAGCTTTTGCTCGGCGAATGTGATGGCGT
CGGAGAGTGGCACCAGTCCCTGCAGGATCAAGGCCAAGAGCTTCAGCAAGTTGTTGTGCAGT
GTAGTTGACTCGCGACGGTTGACCTTGCCGATCACGAACATGTGGTGCTTGAATCTGTTGTAC
TTTTGGATGACCTGGCTCACATCAACGTCCTTGATTTCGCCAGAGATCCAGTAGAACTCCTTGT
TCTTCTTAGCGATGGTCAGCCTTTCCTCGTTCTTGAAGGACAACACGATGAAGTTGTGACCCT
TAACGTTATCGACGTTTTGAGTCAAGTACTGCTCAACGATGTGCATGCTTCCGTCTTCCTTCTT
GACCTTCTTGAGGTTCTCGGCGTTGTTGTTAGAAGCGATGTTGTCGTGGTACTTGTAGTTGTTG
AAGAGCAGGTTAGCGACGGACGAGTACTTGTATGTCAGAGTGCTCTTCTTGTTCACGATGAGG
TTCAGGTTATCAACGACGTACTTGTTGGGAGGGTTGTCCGGGAATTGGACGGACTCCGAGTAC
TTGAGGATCTGGGAAACGTATGGCGAGACGAAGAAGTTGTTAGAGGCGGTTTCGATCTCGTT
TGATTCCTTGCGGCTCAGCATGATGGGCAAAGTGAAGAGGTTCTTGTCCTGGTACATCTCGTA
CAACTTCGACAAGAGGAATCCACACTTACGCTCGCCGAGTGATTGCAGCAAGGTCACGAAGG
TTGTCTGAGCGTAGTACATATCGAGGTTGAAGTAGGAGGTCAGAGCGGCCTTGAATGTGTGGT
GGACGTCCACGAAGTGACACTTCTTGCAGTTTTGAGCGGCGGTCTCATCGTTGCAGACGTCC
TGTGAGTGTGGGATTTCGATGCCAGTCTCCTTAACCAGGTTGTAGCTGATCATGAAGCGGATC
TTATCGAAGGTCACAACGAACACACGGTTGTCAACCATGTAGTAGTTGTTTGTGTACTCGTAG
ACCACGTTAGAGACGTACTTGGCGAAGATGATTTCGAAAGGCTTCACCTCGGACTTCTTAACG
ACGAACATGTAGTAACCGGTTTCAGACATGTGATCGGAGAATCTGTTCGAGTTGTAGTCGTTA

TCGTCGAACCTCATCAGGTAGGGGGCGAAGTCGTTTGTGAAGTAGTGAGTGATCTCCTGGGT
GGAAGCCACTGTACAGATGTTAGTGTTGTGGTTGATGGTTTGTTCCAGTGTAGCGCATGACTG
GATGGTGCTCTTCTTGTACTTAGGTCTCAACTTGATCTTGTTGAATTGACCCACAACTCCCTGG
GAGTTATCCAGGTACTCGTCCAACTTCCTCTTGGTGCCTGTGGCCGACGGCTGGTTGACACCA
GCAGAGTGTTCGAAGGACTCGGCGTGGTAAGCTGAGCTAGGAGAGGGTTGTTCGACCACTG
GCTGTTCGAGTGACTCGCTGTAGTAGGCAGAGGACACAGCTTCCTCCAGGTTGTCAGTGGTC
TTGAGCAGACACTCCACCAAATCGTTGTCAGTGAGCGAGTTAACTGAGGCGAGGAAGTTGCT
AGCGGCAGCGGTTTCAGAGTCGGAGATGACAGTATCAGCTCCGTCCGGGGTTGGGTGGTTGT
AGTAAGACAAGTAATCGTTAGGTTGCTTGTCGCAGAACTCCGAGTATGAGTTGTCGAAGCTAG
CACGAGAGGGAGTGCTGGCAGAGGTGTAGGAAGCGTTGAAGTTGATTTGAGTCATGGTGACC
TGGTTGTTCACGATCTTATCGCCACCTGTGTCCACCTGCAGTTGCTGGGCCTCAGCGCAGGCT
GAAGTGGCCTCACAGGAGTAGGGGCTGGAAGCACAGTTGGAAGTCATGATGTTTTCTTGGAC
GTTCAGGACGTGGCTGGATGTACGGATCATGCGCCCGATGGTGGGACGGTATGAATAATCCGG
AATATTTATAGGTTTTTTTATTACAAAACTGTTACGAAAACAGTAAAATACTTATTTATTTGCGA
GATGGTTATCATTTTAATTATCTCCATGATCGGATCTCCTAGGCTCAAGCAGTGATCAGATCCAG
ACATGATAAGATACATTGATGAGTTTGGACAAACCACAACTAGAATGCAGTGAAAAAAATGCT
TTATTTGTGAAATTTGTGATGCTATTGCTTTATTTGTAACCATTATAAGCTGCAATAAACAAGTT
AACAACAACAATTGCATTTGTCTAGTACTTCTCGACGGTACCGCATGCTATGCATCAGCTGCTA
GCACCATGGCTCGAGTTACAGATTACGAGTCAGGTATCTGGTGCCAATGGGGCGAGGCTCTGA
ATACACGCCATTAGTGTCCACAGTAAAGTCCACATTAACAGACTTGTTGTAGTTGGAAGTGTA
CTGAATTTCGGGATTCCAGCGTTTGCTGTTTTCCTTCTGCAGCTCCCACTCGATCTCCACGCTG
ACCTGTCCCGTGGAGTACTGTGTGATGAAGGAAGCAAACTTTGCCGCACTGAAGGTGGTCGA
AGGATTCGCAGGTACCGGGGTGTTCTTGATGAGAATCTGTGGAGGAGGGTGTTTAAGTCCGA
ATCCACCCATGAGGGGAGAGGGGTGAAAATGTCCGTCCGTGTGTGGAATCTTTGCCCAGATG
GGCCCCTGAAGGTACACATCTCTGTCCTGCCAGACCATGCCTGGAAGAACGCCTTGTGTGTTG
ACATCTGCGGTAGCTGCTTGTCTGTTGCCTCTCTGGAGGTTGGTAGATACAGAACCATACTGCT
CCGTAGCCACGGGATTGGTTGTCCTGATTTCCTCTTCGTCTGTAATCATGACCTTTTCAATGTC
CACATTTGTTTTCTCTGAGCCTTGCTTCCCAAAGATGAGAACCCCGCTCTGAGGAAAAAACTT
TTCTTCATCGTCCTTGTGGCTTGCCATCGCCGGGCCCGGATTCACCAGAGAGTCTCTGCCATTG
AGGTGGTACTTGGTAGCTCCAGTCCACGAGTATTCACTGTTGTTGTTATCCGCAGATGTCTTTG
ATACTCGCTGCTGGCGGTAACAGGGTCCAGGAAGCCAGTTCCTAGACTGGTCCCGAATGTCA
CTCGCTCCGGCCTGAGAAAACTGAAGCCTTGACTGCGTGGTGGTTCCACTTGGAGTGTTTGT
TCTGCTCAAGTAATACAGGTACTGGTCGATGAGAGGATTCATGAGACGGTCCAGACTCTGGCT
GTGAGCGTAGCTGCTGTGGAAAGGAACGTCCTCAAAAGTGTAGCTGAAGGTAAAGTTGTTTC

CGGTACGCAGCATCTGAGAAGGAAAGTACTCCAGGCAGTAAAATGAAGAGCGTCCTACTGCC
TGACTCCCGTTGTTCAGGGTGAGGTATCCATACTGTGGCACCATGAAGACGTCTGCTGGGAAC
GGCGGGAGGCATCCTTGATGCGCCGAGCCGAGGACGTACGGGAGCTGGTACTCCGAGTCAGT
AAACACCTGAACCGTGCTGGTAAGGTTATTGGCAATCGTCGTCGTACCGTCATTCTGCGTGAC
CTCTTTGACTTGAATGTTAAAGAGCTTGAAGTTGAGTCTCTTGGGTCGGAATCCCCAGTTGTT
GTTGATGAGTCTTTGCCAGTCACGTGGTGAAAAGTGGCAGTGGAATCTGTTGAAGTCAAAAT
ACCCCCAAGGGGTGCTGTAGCCAAAGTAGTGATTGTCGTTCGAGGCTCCTGATTGGCTGGAA
ATTTGTTTGTAGAGGTGGTTGTTGTAGGTGGGCAGGGCCCAGGTTCGGGTGCTGGTGGTGATG
ACTCTGTCGCCCATCCATGTGGAATCGCAATGCCAATTTCCCGAGGAATTACCCACTCCGTCG
GCGCCCTCGTTATTGTCTGCCATTGGTGCGCCACTGCCTGTAGCCATCGTATTAGTTCCCAGAC
CAGAGGGGGCTGCTGGTGGCTGTCCGAGAGGCTGGGGGTCAGGTACTGAGTCTGCGTCTCCA
GTCTGACCAAAATTCAATCTTTTTCTTGCAGGCTGCTGGCCCGCCTTTCCGGTTCCCGAGGAG
GAGTCTGGCTCCACAGGAGAGTGCTCTACCGGCCTCTTTTTTCCCGGAGCCGTCTTAACAGGT
TCCTCAACCAGGCCCAGAGGTTCAAGAACCCTCTTTTTCGCCTGGAAGACTGCTCGTCCGAG
GTTGCCCCCAAAAGACGTATCTTCTTTAAGGCGCTCCTGGAACTCCGCGTCGGCGTGGTTGTA
CTTGAGGTAAGGGTTGTCTCCGCTGTCGAGCTGCCGGTCGTAGGCTTTGTCGTGCTCGAGGGC
AGCGGCGTCTGCCTCGTTCACCGGCTCTCCCTTGTCCAGTCCGTTGAAGGGTCCGAGGTACTT
GTACCCGGGAAGCACAAGGCCCCTGCTGTCGTCCTTATGCCGCTCTGCGGGCTTTGGTGGTGG
TGGGCCGGGTTTGAGCTTCCACCACTGTCTTATTCCTTCAGAGAGAGTGTCCTCGAGCCAATC
GGGTAGATAACCGTCGGCAGCCGTGGCGGCTCAAGTCTTCGTCGAGTGATTGTAAATAAAATG
TAATTTACAGTATAGTATTTTAATTAATATACAAATGATTTGATAATAATTCTTATTTAACTATAATA
TATTGTGTTGGGTTGAATTAAAGGTCCGTATCTCCAGACTGCAGTTTAAATTGTGTAATTTATGT
AGCTGTAATTTTTACCTTATTAATATTTTTTACGCTTTGCATTCGACGACTGAACTCCCAAATATA
TGTTTAACTCGTCTTGGTCGTTTGAATTTTTGTTGCTGTGTTTCCTAATATTTTCCATCACCTTA
AATATGTTATTGTAATCCTCAATGTTGAACTTGCAATTGGACACGGCATAGTTTTCCATAGTCGT
GTAAAACATGGTATTGGCTGCATTGTAATACATCCGACTGAGCGGGTACGGATCTATGTGTTTG
AGCAGCCTGTTCAAAAACTCTGCATCGTCGCAAAACGGAATTTGGTCACGATCCCGGTCCGA
AGCGCGCGGAATTCAAAGCGCTACGTCAGCGACGTCATCGATTATTGCTCCAATACTAGGGTT
TTGTTTTACTATCTGTTCTGCGGTCCACACATCTTCTTCGCTAGTCACGTACGCAAACAACTCG
GGCCGCAAAGGTGTTTTAAAACATATCAGATTAGAATCTTTTATAACTTGGCCGCATTGTAAAT
AGTTGTGCCAACGCGCGGGAAACATTTTACACGTGCTTTCAATCTTATCTCATAAACGCAACT
AAATATGATCCTTCAACTCGCTTTACGAGTAGAATTCTACTTGTAACGCACGATTAATTATGACT
CATAAGCTGATGTCATGTTTTGCACACGGCTCATAACCGAACTCGCTTTACGAGTAGAATTCTA
CGCGTAAAACACAATCAAGTATGAGTCATAAGCTGATGTCATGTTTTGCACACGGCTCATAAC

CGAACTGGCTTTACGAGTAGAATTCTACTTGTAACGCACGATCAGTGGATGATGTCATAAACTT
ATCTCATGTTTTGCACACGGCTCATAACTTGAGTCATAAGCTGATATCAGAAGATTATTAACGT
GTTTAGGTATGACTCATTTGTTTTCAAAACTGAACTCGCTTTACGGATAGAATTCTACTTGTAA
AACACAATCAGGGGATGATGTCATTATACAAATGATGTCATTTGTTTTTCAAAACTAAACTCGC
TTTACGGGTAGAATTCTACTTGTAAAACACAATCGAGGGATAATGTCATTTGTTATTCTCAAATA
TGATGTCATTTAAATTTTTATTATGCAAGAATTGTATGTTGCTGTGTCATTTATGAATGTTTTATC
GACAACTTGTTTATTTGTTTCATTGACTTGTTGTTGTTCGTGATTATGATTAATTATAAATCTTTT
GTTGGGGTCAACATCGATGTATACGCCGCTACAGGGCGCGTACTATGGTTGCTTTGACGTATGC
GGTGTGAAATACCGCACAGATGCGTAAGGAGAAAATACCGCATCAGGCGCCCCTGCAGGCAG
CTGCGCGCTCGCTCGCTCACTGAGGCCGCCCGGGCAAAGCCCGGGCGTCGGGCGACCTTTGG
TCGCCCGGCCTCAGTGAGCGAGCGAGCGCGCAGAGAGGGAGTGGCCAACTCCATCACTAGG
GGTTCCTACGCGTTAGTTATTAATAGTAATCAATTACGGGGTCATTAGTTCATAGCCCATATATG
GAGTTCCGCGTTACATAACTTACGGTAAATGGCCCGCCTGGCTGACCGCCCAACGACCCCCGC
CCATTGACGTCAATAATGACGTATGTTCCCATAGTAACGCCAATAGGGACTTTCCATTGACGTC
AATGGGTGGAGTATTTACGGTAAACTGCCCACTTGGCAGTACATCAAGTGTATCATATGCCAAG
TACGCCCCCTATTGACGTCAATGACGGTAAATGGCCCGCCTGGCATTATGCCCAGTACATGACC
TTATGGGACTTTCCTACTTGGCAGTACATCTACGTATTAGTCATCGCTATTACCATGGTGATGCG
GTTTTGGCAGTACATCAATGGGCGTGGATAGCGGTTTGACTCACGGGGATTTCCAAGTCTCCA
CCCCATTGACGTCAATGGGAGTTTGTTTTGGCACCAAAATCAACGGGACTTTCCAAAATGTCG
TAACAACTCCGCCCCATTGACGCAAATGGGCGGTAGGCGTGTACGGTGGGAGGTCTATATAAG
CAGAGCTGGTTTAGTGAACCGTCAGATCCGCTAGCGCTACCGGTCGCCACCATGGTGAGCAA
GGGCGAGGAGCTGTTCACCGGGGTGGTGCCCATCCTGGTCGAGCTGGACGGCGACGTAAAC
GGCCACAAGTTCAGCGTGTCCGGCGAGGGCGAGGGCGATGCCACCTACGGCAAGCTGACCC
TGAAGTTCATCTGCACCACCGGCAAGCTGCCCGTGCCCTGGCCCACCCTCGTGACCACCCTG
ACCTACGGCGTGCAGTGCTTCAGCCGCTACCCCGACCACATGAAGCAGCACGACTTCTTCAA
GTCCGCCATGCCCGAAGGCTACGTCCAGGAGCGCACCATCTTCTTCAAGGACGACGGCAACT
ACAAGACCCGCGCCGAGGTGAAGTTCGAGGGCGACACCCTGGTGAACCGCATCGAGCTGAA
GGGCATCGACTTCAAGGAGGACGGCAACATCCTGGGGCACAAGCTGGAGTACAACTACAAC
AGCCACAACGTCTATATCATGGCCGACAAGCAGAAGAACGGCATCAAGGTGAACTTCAAGAT
CCGCCACAACATCGAGGACGGCAGCGTGCAGCTCGCCGACCACTACCAGCAGAACACCCCCA
TCGGCGACGGCCCCGTGCTGCTGCCCGACAACCACTACCTGAGCACCCAGTCCGCCCTGAGC
AAAGACCCCAACGAGAAGCGCGATCACATGGTCCTGCTGGAGTTCGTGACCGCCGCCGGGAT
CACTCTCGGCATGGACGAGCTGTACAAGTAGGTCGACGTTTATTTGTGAAATTTGTGATGCTAT
TGCTTTATTTGTAACCATCTAGCTTTATTTGTGAAATTTGTGATGCTATTGCTTTATTTGTAACCA

TTATAAGCTGCAATAAACAAGTTAACAACAACAATTGCATTCATTTTATGTTTCAGGTTCAGGG
GGAGATGTGGGAGGTTTTTTAAAGTTTAAACAGGAACCCCTAGTGATGGAGTTGGCCACTCCC
TCTCTGCGCGCTCGCTCGCTCACTGAGGCCGGGCGACCAAAGGTCGCCCGACGCCCGGGCGG
CCTCAGTGAGCGAGCGAGCGCGCAGCTGCCTGCAGGACATGTGAGCAAAAGGCCAGCAAAA
GGCCAGGAACCGTAAAAAGGCCGCGTTGCTGGCGTTTTTCCATAGGCTCCGCCCCCCTGACG
AGCATCACAAAAATCGACGCTCGCGGCCGCTCTAGAGATCATGGAGATAATTAAAATGATAAC
CATCTCGCAAATAAATAAGTATTTTACTGTTTTCGTAACAGTTTTGTAATAAAAAAACCTATAAA
TATTCCGGATTATTCATACCGTCCCACCATCGGGCGCGGATCCGCCGCCCTGGCGGGGTTTTAC
GAGATTGTGATTAAGGTCCCCAGCGACCTTGACGAGCATCTGCCCGGCATTTCTGACAGCTTT
GTGAACTGGGTGGCCGAGAAGGAGTGGGAGTTGCCGCCAGATTCTGACTTGGATCTGAATCT
GATTGAGCAGGCACCCCTGACCGTGGCCGAGAAGCTGCAGCGCGACTTTCTGACGGAGTGG
CGCCGTGTGAGTAAGGCCCCGGAGGCCCTTTTCTTTGTGCAATTTGAGAAGGGAGAGAGCTA
CTTCCACTTACACGTGCTCGTGGAAACCACCGGGGTGAAATCCTTAGTTTTGGGACGTTTCCT
GAGTCAGATTCGCGAAAAACTGATTCAGAGAATTTACCGCGGGATCGAGCCGACTTTGCCAA
ACTGGTTCGCGGTCACAAAGACCAGAAACGGCGCCGGAGGCGGGAACAAGGTGGTGGACG
AGTGCTACATCCCCAATTACTTGCTCCCCAAAACCCAGCCTGAGCTCCAGTGGGCGTGGACTA
ATTTAGAACAGTATTTAAGCGCCTGTTTGAATCTCACGGAGCGTAAACGGTTGGTGGCGCAGC
ATCTGACGCACGTGTCGCAGACGCAGGAGCAGAACAAAGAGAATCAGAATCCCAATTCTGAC
GCGCCGGTGATCAGATCAAAAACTTCAGCCAGGTACATGGAGCTGGTCGGGTGGCTCGTGGA
CAAGGGGATTACCTCGGAGAAGCAGTGGATACAGGAGGACCAGGCCTCATACATCTCCTTCA
ATGCGGCCTCCAACTCGCGGTCCCAAATCAAGGCTGCCTTGGACAATGCGGGAAAGATTATG
AGCCTGACTAAAACCGCCCCCGACTACCTGGTGGGCCAGCAGCCCGTGGAGGACATTTCCAG
CAATCGGATTTATAAAATTTTGGAACTAAACGGGTACGATCCCCAATATGCGGCTTCCGTCTTT
CTGGGATGGGCCACGAAAAAGTTCGGCAAGAGGAACACCATCTGGCTGTTTGGGCCTGCAAC
TACCGGGAAGACCAACATCGCGGAGGCCATAGCCCACACTGTGCCCTTCTACGGGTGCGTAA
ACTGGACCAATGAGAACTTTCCCTTCAACGACTGTGTGGACAAGATGGTGATCTGGTGGGAG
GAGGGGAAGATGACCGCCAAGGTCGTGGAGTCGGCCAAAGCCATTCTCGGAGGAAGCAAGG
TGCGCGTGGACCAGAAATGCAAGTCCTCGGCCCAGATAGACCCGACTCCCGTGATCGTCACC
TCCAACACCAACATGTGCGCCGTGATTGACGGGAACTCAACGACCTTCGAACACCAGCAGCC
GTTGCAAGACCGGATGTTCAAATTTGAACTCACCCGCCGTCTGGATCATGACTTTGGGAAGGT
CACCAAGCAGGAAGTCAAAGACTTTTTCCGGTGGGCAAAGGATCACGTGGTTGAGGTGGAG
CATGAATTCTACGTCAAAAAGGGTGGAGCCAAGAAAAGACCCGCCCCCAGTGACGCAGATAT
AAGTGAGCCCAAACGGGTGCGCGAGTCAGTTGCGCAGCCATCGACGTCAGACGCGGAAGCA
TCGATCAACTACGCAGACAGGTACCAAAACAAATGTTCTCGTCACGTGGGCATGAATCTGATG

CTGTTTCCCTGCAGACAATGCGAGAGAATGAATCAGAATTCAAATATCTGCTTCACTCACGGA
CAGAAAGACTGTTTAGAGTGCTTTCCCGTGTCAGAATCTCAACCCGTTTCTGTCGTCAAAAAG
GCGTATCAGAAACTGTGCTACATTCATCATATCATGGGAAAGGTGCCAGACGCTTGCACTGCC
TGCGATCTGGTCAATGTGGATTTGGATGACTGCATCTTTGAACAATAATCTAGAGCCTGCAGTC
TCGACAAGCTTGTCGAGAAGTACTAGAGGATCATAATCAGCCATACCACATTTGTAGAGGTTTT
ACTTGCTTTAAAAAACCTCCCACACCTCCCCCTGAACCTGAAACATAAAATGAATGCAATTGT
TGTTGTTAACTTGTTTATTGCAGCTTATAATGGTTACAAATAAAGCAATAGCATCACAAATTTCA
CAAATAAAGCATTTTTTTCACTGCATTCTAGTTGTGGTTTGTCCAAACTCATCAATGTATCTTAT
CATGTCTGGATCTGATCACTGCTTGAGCCTAGGAGATCCGAACCAGATAAGTGAAATCTAGTT
CCAAACTATTTTGTCATTTTTAATTTTCGTATTAGCTTACGACGCTACACCCAGTTCCCATCTATT
TTGTCACTCTTCCCTAAATAATCCTTAAAAACTCCATTTCCACCCCTCCCAGTTCCCAACTATTT
TGTCCGCCCACAGCGGGGCATTTTTCTTCCTGTTATGTTTTTAATCAAACATCCTGCCAACTCC
ATGTGACAAACCGTCATCTTCGGCTACTTT

The two recombinant vectors were transformed into DH10Bac competent cells (Invitrogen) for blue-white screening, respectively. The white positive clones were picked and cultured, and the bacmids were extracted using PureLink^{™} HiPure Plasmid DNA Purification Kits (Invitrogen). The two types of bacmids were transfected into adherent Sf9 cells (Gibco), and the primary recombinant baculoviruses were harvested 3 days later and amplified to the P2 generation. Finally, the recombinant baculoviruses of P2 generation were named BV-Cap-ITR-Rep and BV-IE-hr1Cap-ITR-Rep, respectively, and the virus titers were determined by TCID50 method. For details of this part, refer to the Bac-to-Bac Expression System Operation Manual (Invitrogen).

### Example 2. Packaging and titer determination of rAAV2

The two types of recombinant baculoviruses of P2-generation were used to infect 25 mL of suspension Sf9 cells (density 3.0E+06 cell/mL) at MOI=0.1, and the cells were harvested after 4 days. 5 mL of TNT lysis solution (20 mM Tris-HCl, pH 7.5, 150 mM NaCl, 1% Triton X-100, 10 mmol/L MgCh) was used and incubated at room temperature for 1 h to lyse the cells. Add nuclease at a final concentration of 50 U/mL to digest the cell lysate at 37°C for 2 h, take samples and use proteinase K (Tiangen) to digest the capsid at 56°C for 1 h, and determine the titer by qPCR. The detection steps are as follows:
The standard used in the quantitative PCR is the pAAV-EGFP plasmid linearized by single restriction enzyme digestion with PvuI-HF (NEB) (the plasmid sequence is shown in SEQ ID NO: 22).

SEQ ID NO.22：
ACATGTGAGCAAAAGGCCAGCAAAAGGCCAGGAACCGTAAAAAGGCCGCGTTGCTGGCGTT

TTTCCATAGGCTCCGCCCCCCTGACGAGCATCACAAAAATCGACGCTCAAGTCAGAGGTGGC
GAAACCCGACAGGACTATAAAGATACCAGGCGTTTCCCCCTGGAAGCTCCCTCGTGCGCTCTC
CTGTTCCGACCCTGCCGCTTACCGGATACCTGTCCGCCTTTCTCCCTTCGGGAAGCGTGGCGC
TTTCTCATAGCTCACGCTGTAGGTATCTCAGTTCGGTGTAGGTCGTTCGCTCCAAGCTGGGCTG
TGTGCACGAACCCCCCGTTCAGCCCGACCGCTGCGCCTTATCCGGTAACTATCGTCTTGAGTC
CAACCCGGTAAGACACGACTTATCGCCACTGGCAGCAGCCACTGGTAACAGGATTAGCAGAG
CGAGGTATGTAGGCGGTGCTACAGAGTTCTTGAAGTGGTGGCCTAACTACGGCTACACTAGAA
GGACAGTATTTGGTATCTGCGCTCTGCTGAAGCCAGTTACCTTCGGAAAAAGAGTTGGTAGCT
CTTGATCCGGCAAACAAACCACCGCTGGTAGCGGTGGTTTTTTTGTTTGCAAGCAGCAGATTA
CGCGCAGAAAAAAAGGATCTCAAGAAGATCCTTTGATCTTTTCTACGGGGTCTGACGCTCAGT
GGAACGAAAACTCACGTTAAGGGATTTTGGTCATGAGATTATCAAAAAGGATCTTCACCTAGA
TCCTTTTAAATTAAAAATGAAGTTTTAAATCAATCTAAAGTATATATGAGTAAACTTGGTCTGAC
AGTTACCAATGCTTAATCAGTGAGGCACCTATCTCAGCGATCTGTCTATTTCGTTCATCCATAGT
TGCCTGACTCCCCGTCGTGTAGATAACTACGATACGGGAGGGCTTACCATCTGGCCCCAGTGC
TGCAATGATACCGCGAGACCCACGCTCACCGGCTCCAGATTTATCAGCAATAAACCAGCCAGC
CGGAAGGGCCGAGCGCAGAAGTGGTCCTGCAACTTTATCCGCCTCCATCCAGTCTATTAATTG
TTGCCGGGAAGCTAGAGTAAGTAGTTCGCCAGTTAATAGTTTGCGCAACGTTGTTGCCATTGC
TACAGGCATCGTGGTGTCACGCTCGTCGTTTGGTATGGCTTCATTCAGCTCCGGTTCCCAACGA
TCAAGGCGAGTTACATGATCCCCCATGTTGTGCAAAAAAGCGGTTAGCTCCTTCGGTCCTCCG
ATCGTTGTCAGAAGTAAGTTGGCCGCAGTGTTATCACTCATGGTTATGGCAGCACTGCATAATT
CTCTTACTGTCATGCCATCCGTAAGATGCTTTTCTGTGACTGGTGAGTACTCAACCAAGTCATT
CTGAGAATAGTGTATGCGGCGACCGAGTTGCTCTTGCCCGGCGTCAATACGGGATAATACCGC
GCCACATAGCAGAACTTTAAAAGTGCTCATCATTGGAAAACGTTCTTCGGGGCGAAAACTCTC
AAGGATCTTACCGCTGTTGAGATCCAGTTCGATGTAACCCACTCGTGCACCCAACTGATCTTC
AGCATCTTTTACTTTCACCAGCGTTTCTGGGTGAGCAAAAACAGGAAGGCAAAATGCCGCAA
AAAAGGGAATAAGGGCGACACGGAAATGTTGAATACTCATACTCTTCCTTTTTCAATATTATTG
AAGCATTTATCAGGGTTATTGTCTCATGAGCGGATACATATTTGAATGTATTTAGAAAAATAAAC
AAATAGGGGTTCCGCGCACATTTCCCCGAAAAGTGCCACCTGACGTCTAAGAAACCATTATTA
TCATGACATTAACCTATAAAAATAGGCGTATCACGAGGCCCTTTCGTCTCGCGCGTTTCGGTGA
TGACGGTGAAAACCTCTGACACATGCAGCTCCCGGAGACGGTCACAGCTTGTCTGTAAGCGG
ATGCCGGGAGCAGACAAGCCCGTCAGGGCGCGTCAGCGGGTGTTGGCGGGTGTCGGGGCTG
GCTTAACTATGCGGCATCAGAGCAGATTGTACTGAGAGTGCACCATAAAATTGTAAACGTTAAT
ATTTTGTTAAAATTCGCGTTAAATTTTTGTTAAATCAGCTCATTTTTTAACCAATAGGCCGAAAT
CGGCAAAATCCCTTATAAATCAAAAGAATAGCCCGAGATAGGGTTGAGTGTTGTTCCAGTTTG

GAACAAGAGTCCACTATTAAAGAACGTGGACTCCAACGTCAAAGGGCGAAAAACCGTCTATC
AGGGCGATGGCCCACTACGTGAACCATCACCCAAATCAAGTTTTTTGGGGTCGAGGTGCCGT
AAAGCACTAAATCGGAACCCTAAAGGGAGCCCCCGATTTAGAGCTTGACGGGGAAAGCCGGC
GAACGTGGCGAGAAAGGAAGGGAAGAAAGCGAAAGGAGCGGGCGCTAGGGCGCTGGCAAG
TGTAGCGGTCACGCTGCGCGTAACCACCACACCCGCCGCGCTTAATGCGCCGCTACAGGGCG
CGTACTATGGTTGCTTTGACGTATGCGGTGTGAAATACCGCACAGATGCGTAAGGAGAAAATA
CCGCATCAGGCGCCCCTGCAGGCAGCTGCGCGCTCGCTCGCTCACTGAGGCCGCCCGGGCAA
AGCCCGGGCGTCGGGCGACCTTTGGTCGCCCGGCCTCAGTGAGCGAGCGAGCGCGCAGAGA
GGGAGTGGCCAACTCCATCACTAGGGGTTCCTACGCGTTAGTTATTAATAGTAATCAATTACGG
GGTCATTAGTTCATAGCCCATATATGGAGTTCCGCGTTACATAACTTACGGTAAATGGCCCGCCT
GGCTGACCGCCCAACGACCCCCGCCCATTGACGTCAATAATGACGTATGTTCCCATAGTAACG
CCAATAGGGACTTTCCATTGACGTCAATGGGTGGAGTATTTACGGTAAACTGCCCACTTGGCA
GTACATCAAGTGTATCATATGCCAAGTACGCCCCCTATTGACGTCAATGACGGTAAATGGCCCG
CCTGGCATTATGCCCAGTACATGACCTTATGGGACTTTCCTACTTGGCAGTACATCTACGTATTA
GTCATCGCTATTACCATGGTGATGCGGTTTTGGCAGTACATCAATGGGCGTGGATAGCGGTTTG
ACTCACGGGGATTTCCAAGTCTCCACCCCATTGACGTCAATGGGAGTTTGTTTTGGCACCAAA
ATCAACGGGACTTTCCAAAATGTCGTAACAACTCCGCCCCATTGACGCAAATGGGCGGTAGG
CGTGTACGGTGGGAGGTCTATATAAGCAGAGCTGGTTTAGTGAACCGTCAGATCCGCTAGCGC
TACCGGTCGCCACCATGGTGAGCAAGGGCGAGGAGCTGTTCACCGGGGTGGTGCCCATCCTG
GTCGAGCTGGACGGCGACGTAAACGGCCACAAGTTCAGCGTGTCCGGCGAGGGCGAGGGCG
ATGCCACCTACGGCAAGCTGACCCTGAAGTTCATCTGCACCACCGGCAAGCTGCCCGTGCCC
TGGCCCACCCTCGTGACCACCCTGACCTACGGCGTGCAGTGCTTCAGCCGCTACCCCGACCA
CATGAAGCAGCACGACTTCTTCAAGTCCGCCATGCCCGAAGGCTACGTCCAGGAGCGCACCA
TCTTCTTCAAGGACGACGGCAACTACAAGACCCGCGCCGAGGTGAAGTTCGAGGGCGACAC
CCTGGTGAACCGCATCGAGCTGAAGGGCATCGACTTCAAGGAGGACGGCAACATCCTGGGGC
ACAAGCTGGAGTACAACTACAACAGCCACAACGTCTATATCATGGCCGACAAGCAGAAGAAC
GGCATCAAGGTGAACTTCAAGATCCGCCACAACATCGAGGACGGCAGCGTGCAGCTCGCCGA
CCACTACCAGCAGAACACCCCCATCGGCGACGGCCCCGTGCTGCTGCCCGACAACCACTACC
TGAGCACCCAGTCCGCCCTGAGCAAAGACCCCAACGAGAAGCGCGATCACATGGTCCTGCTG
GAGTTCGTGACCGCCGCCGGGATCACTCTCGGCATGGACGAGCTGTACAAGTAGGTCGACGT
TTATTTGTGAAATTTGTGATGCTATTGCTTTATTTGTAACCATCTAGCTTTATTTGTGAAATTTGT
GATGCTATTGCTTTATTTGTAACCATTATAAGCTGCAATAAACAAGTTAACAACAACAATTGCA
TTCATTTTATGTTTCAGGTTCAGGGGGAGATGTGGGAGGTTTTTTAAAGTTTAAACAGGAACC
CCTAGTGATGGAGTTGGCCACTCCCTCTCTGCGCGCTCGCTCGCTCACTGAGGCCGGGCGACC

AAAGGTCGCCCGACGCCCGGGCGGCCTCAGTGAGCGAGCGAGCGCGCAGCTGCCTGCAGG

The sequences of the primers used in quantitative PCR are:
ITR Forward primer 5'-GGAACCCCTAGTGATGGAGTT-3' (SEQ ID NO: 2)
ITR Reverse primer 5'-CGGCCTCAGTGAGCGA-3' (SEQ ID NO: 3)

The running program of quantitative PCR was: 95°C for 60 s, (95°C for 15 s, 60°C for 30 s, 40 cycles). After plotting the standard curve according to the Ct value obtained by quantitative PCR and the concentrations of the standard, the titer of the sample was calculated. The yields of rAAVs packaged using BV-Cap-ITR-Rep and BV-IE-hr1Cap-ITR-Rep were determined to be 2.39E+04 VG/cell (equivalent to 7.18 E+10 VG/mL) and 8.67 E+05 VG/cell (equivalent to 2.60 E+12 VG/mL), respectively.

### Example 3. Comparison of the expression of EGFP in the process of cell infection and the expression of Cap proteins in the cell lysates

After 48 h, 72 h, and 96 h of infection, the cells infected by the two baculoviruses were examined under an inverted fluorescence microscope. The optimized recombinant baculovirus can greatly reduce the expression of the target gene (the target gene in this example is EGFP) (Figure 5). The reduction of the expression of the target gene (EGFP) seems to weaken the interference to rAAV packaging in cells, making it more conducive to the expression of rAAV packaging-related structural and functional proteins.

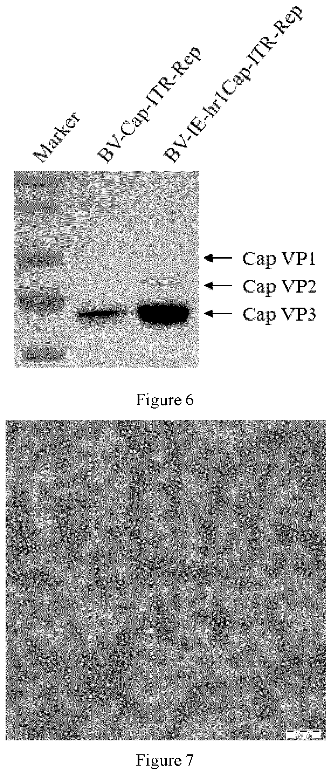

The same amount of cell lysates was assayed by Western blotting using Cap protein antibody (Progen) (Figure 6). The results showed that after the optimized recombinant baculovirus infected the cells, the expression levels of Cap proteins in the cells were significantly increased.

### Example 4. Electron microscopy examination of purified rAAV2 and infectivity determination

POROS^{™} CaptureSelect^{™} affinity chromatography media (Thermo) was used to purify rAAV2 from the cell lysate, and its titer was determined by fluorescent quantitative PCR (the determination method is the same as in Example 2). The purified rAAV2 was subject to negative staining treatment and examined using transmission electron microscopy (Figure 7). The genome-packaged rAAV2 was solid particles, and the center of defective rAAV particles without nucleic acid was stained dark. Overall, the morphology of the purified rAAV2 is good, and the empty capsid rate is significantly reduced (~3%) compared with those obtained using traditional method (Benskey et al., 2016), suggesting that the optimized baculovirus vector construct significantly reduces the proportion of empty capsid viruses in the packaging product, which would significantly reduce the burden on downstream steps such as removal of empty capsid viruses.

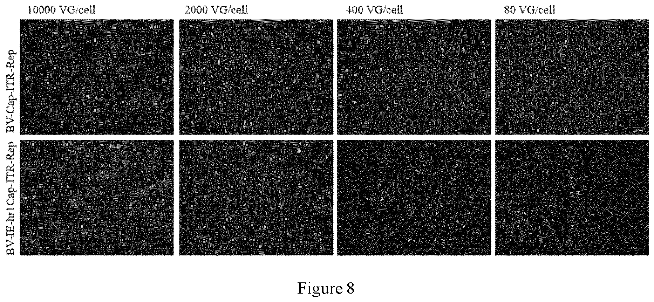

The purified rAAV2 was serially diluted and used to infect 293T cells cultured on 48-well plate (5.0 E+04 cells/well) at MOI=10000, 2000, 400, and 80, respectively. Two days after infection, the expression of EGFP was examined by fluorescence microscopy. The experimental results show that the rAAV2 prepared using this system has high *in vitro* infection activity (Figure 8).

The above examples are intended to illustrate the disclosed embodiments of the present invention, and should not be construed as limiting the present invention. In addition, various modifications set forth herein, as well as changes in the method of the invention, will be obvious to those skilled in the art without departing from the scope and spirit of the invention. Although the invention has been specifically described in connection with various specific preferred embodiments of the invention, it should be understood that the invention should not be limited to these specific embodiments. In fact, various modifications as mentioned above which are obvious to those skilled in the art to obtain the invention should be included in the scope of the present invention.

## Claims

1. A nucleic acid construct, wherein the nucleic acid construct comprises: AAV elements and a polynucleotide encoding an IE protein, and the AAV elements comprise a polynucleotide encoding Cap proteins, a polynucleotide encoding Rep proteins, and AAV cis-acting elements.

2. The nucleic acid construct according to claim 1, wherein the nucleic acid construct further comprises a polynucleotide encoding a recombination homologous region of baculovirus.

3. The nucleic acid construct according to claim 2, wherein the IE protein is encoded by one or more of Acie0, Acie01, and Acie02 genes, and/or, the recombination homologous region of baculovirus is selected from one or more of hr1, hr2, hr3, hr4, and hr5.

4. The nucleic acid construct according to claim 1, wherein the nucleic acid construct further comprises a promoter of an IE protein-coding gene, and the promoter of the IE protein-coding gene is selected from one or more of Gp64, pH, p6.9, and p10.

5. The nucleic acid construct according to claim 1, wherein the nucleic acid construct further comprises a baculovirus promoter connected to a recombination homologous region of baculovirus, and the baculovirus promoter is preferably one or more of pH, Gp64, p6.9, and p10.

6. The nucleic acid construct according to claim 1, wherein the AAV cis-acting elements are selected from ITR sequences.

7. The nucleic acid construct according to claim 1, wherein the nucleic acid construct further comprises an exogenous gene of interest embedded in between AAV elements.

8. The nucleic acid construct according to claim 7, wherein the nucleic acid construct has a structure: IE gene expression cassette-Cap gene expression cassette-ITR-exogenous gene of interest expression cassette-ITR- Rep gene expression cassette.

9. The nucleic acid construct according to claim 1, wherein the nucleotide sequence of the nucleic acid construct is as shown in SEQ ID NO. 1.

10. The nucleic acid construct according to claim 1, wherein the nucleic acid construct is an adeno-associated virus vector or a recombinant baculovirus vector, and the recombinant baculovirus vector is preferably a recombinant baculovirus shuttle vector.

11. A recombinant baculovirus, wherein the recombinant baculovirus is obtained by constructing the nucleic acid construct of any one of claims 1-10 through a baculovirus system, or by jointly constructing the nucleic acid constructs containing any element in the nucleic acid construct of any one of claims 1-10 through the baculovirus system.

12. An adeno-associated virus, wherein the adeno-associated virus is obtained by infecting cells with the recombinant baculovirus of claim 11 and packaging.

13. A cell line, wherein the cell line is a cell line infected with the recombinant baculovirus of claim 11.

14. An adeno-associated virus vector system, wherein the adeno-associated virus vector system comprises a baculovirus system and the nucleic acid construct of any one of claims 1-10.

15. A method for constructing the nucleic acid construct of any one of claims 1-10, wherein the method comprises integrating AAV elements carrying an exogenous gene of interest, a polynucleotide encoding an IE protein, and a polynucleotide encoding a recombination homologous region of baculovirus into a backbone of a baculovirus vector.

16. The method according to claim 15, wherein the method includes one or more of:
1) the AAV elements include a polynucleotide encoding Cap proteins, a polynucleotide encoding Rep proteins, and AAV cis-acting elements, and the AAV cis-acting elements are preferably ITR sequences;
2) The polynucleotide encoding the IE protein is selected from one or more of Acie0, Acie01, and Acie02 genes;
3) The backbone of the baculovirus vector is selected from one of pFastBacdual, pFastBac1, pFastBacHTA, pFastBacHTB, and pFastBacHTC;
4) The recombination homologous region of baculovirus is selected from one or more of hr1, hr2, hr3, hr4, and hr5.

17. A method for producing an adeno-associated virus, wherein the method comprises the step of infecting an insect cell line with the recombinant baculovirus of claim 11.
